# EUROPÄISCHE PATENTSCHRIFT

(11) **EP 1 373 506 B1**
(45) Veröffentlichungstag und Bekanntmachung des Hinweises auf die Patenterteilung: **15.04.2009**
(21) Anmeldenummer: 02719792.0
(22) Anmeldetag: 09.02.2002
(51) Int. Cl.: C12N 15/12, C12N 15/81, C12N 1/18, C07K 14/62, C12Q 1/02, C12Q 1/54

(54) **HEFESTAMM VON SACCHAROMYCES CEREVISIAE MIT FUNKTIONELLER EXPRESSION EINES GLUT-TRANSPORTERS**
YEAST STRAIN OF SACCHAROMYCES CEREVISIAE WITH FUNCTIONAL EXPRESSION OF A GLUT TRANSPORTER
SOUCHE DE LEVURE DE SACCHAROMYCES CEREVISIAE AVEC EXPRESSION FONCTIONNELLE D'UN TRANSPORTEUR GLUT

(30) Priorität: 14.02.2001 DE 10106718
(43) Veröffentlichungstag der Anmeldung: 02.01.2004
(73) Patentinhaber: Sanofi-Aventis Deutschland GmbH, 65929 Frankfurt am Main (DE)
(72) Erfinder: MÜLLER, Günter, 65843 Sulzbach a. Ts. (DE); KOLLER, Klaus-Peter, 65812 Bad Soden (DE); BOLES, Eckhard, 63303 Dreieich (DE); WIECZORKE, Roman, 40627 Düsseldorf (DE); DLUGAI, Silke, 81541 München (DE)
(86) Internationale Anmeldenummer: PCT/EP2002/001373
(87) Internationale Veröffentlichungsnummer: WO 2002/064784

(56) Entgegenhaltungen:
- WIECZORKE R ET AL: "Concurrent knock-out of at least 20 transporter genes is required to block uptake of hexoses in Saccharomyces cerevisiae" FEBS LETTERS, ELSEVIER SCIENCE PUBLISHERS, AMSTERDAM, NL, Bd. 464, Nr. 3, 31. Dezember 1999 (1999-12-31), Seiten 123-128, XP004260734 ISSN: 0014-5793
- KRUCKEBERG A L ET AL: "THE HXT2 GENE OF SACCHAROMYCES-CEREVISIAE IS REQUIRED FOR HIGH-AFFINITY GLUCOSE TRANSPORT" MOLECULAR AND CELLULAR BIOLOGY, Bd. 10, Nr. 11, 1990, Seiten 5903-5913, XP009007277 ISSN: 0270-7306
- KASAHARA TOSHIKO ET AL: "Characterization of rat Glut4 glucose transporter expressed in the yeast Saccharomyces cerevisiae: Comparison with Glut1 glucose transporter." BIOCHIMICA ET BIOPHYSICA ACTA, Bd. 1324, Nr. 1, 1997, Seiten 111-119, XP001121502 ISSN: 0006-3002
- KASAHARA TOSHIKO ET AL: "Expression of the rat GLUT1 glucose transporter in the yeast Saccharomyces cerevisiae." BIOCHEMICAL JOURNAL, Bd. 315, Nr. 1, 1996, Seiten 177-182, XP009002582 ISSN: 0264-6021
- KASAHARA TOSHIKO ET AL: "Studies on the Glut family transporters: Use of the yeast expression system." MEMBRANE PROTEINS: STRUCTURE, FUNCTION AND EXPRESSION CONTROL., 1997, Seiten 201-212, XP001121504 International Symposium, Kyushu University Press;S. Karger AG 7-1-146, Hakozaki, Higashi-ku, Fukuoka 812, Japan; P.O. Box, Allschwilerstrasse 10, CH-4009 Basel, Switzerland ISBN: 3-8055-6465-1
- FUKUMOTO H ET AL: "CLONING AND CHARACTERIZATION OF THE MAJOR INSULIN-RESPONSIVE GLUCOSE TRANSPORTER EXPRESSED IN HUMAN SKELETAL MUSCLE AND OTHER INSULIN-RESPONSIVE TISSUES" JOURNAL OF BIOLOGICAL CHEMISTRY, Bd. 264, Nr. 14, 1989, Seiten 7776-7779, XP002225835 ISSN: 0021-9258
- BIRNBAUM M J ET AL: "CLONING AND CHARACTERIZATION OF A CDNA ENCODING THE RAT BRAIN GLUCOSE-TRANSPORTER PROTEIN" PROCEEDINGS OF THE NATIONAL ACADEMY OF SCIENCES OF USA, NATIONAL ACADEMY OF SCIENCE. WASHINGTON, US, Bd. 83, 1. August 1986 (1986-08-01), Seiten 5784-5788, XP002068497 ISSN: 0027-8424
- KASAHARA TOSHIKO ET AL: "Tryptophan 388 in putative transmembrane segment 10 of the rat glucose transporter Glut1 is essential for glucose transport." JOURNAL OF BIOLOGICAL CHEMISTRY, Bd. 273, Nr. 44, 30. Oktober 1998 (1998-10-30), Seiten 29113-29117, XP001121501 ISSN: 0021-9258
- ASANO T ET AL: "THE ROLE OF N-GLYCOSYLATION OF GLUT1 FOR GLUCOSE TRANSPORT ACTIVITY" JOURNAL OF BIOLOGICAL CHEMISTRY, Bd. 266, Nr. 36, 1991, Seiten 24632-24636, XP002236407 ISSN: 0021-9258

### SEQUENZPROTOKOLL

<110> Aventis Pharma Deutschland GmbH
<120> Hefestamm von Saccharomyces cerevisiae mit funktioneller Espression eines Glut-Transporters
<130> 2001/0002
<140> 10106718.6
   <141> 2001-02-14
<160> 18
<170> PatentIn Ver. 2.1
<210> 1
   <211> 25
   <212> DNA
   <213> Homo sapiens
<400> 1
   ctagagctcg taggaacaat ttcgg 25
<210> 2
   <211> 60
   <212> DNA
   <213> Homo sapiens
<400> 2
   cgactagtgt gatggtgatg gtgatgcatg ttaacttttt gattaaaatt aaaaaaactt 60
<210> 3
   <211> 35
   <212> DNA
   <213> Homo sapiens
<400> 3
   ttaattttaa tcaaaaaatg gagcccagca gcaag 35
<210> 4
   <211> 52
   <212> DNA
   <213> Homo sapiens
<400> 4
   acatgactcg aggtcgacgg tatcgataag cttatcacac ttgggaatca gc 52
<210> 5
   <211> 35
   <212> DNA
   <213> Homo sapiens
<400> 5
   ttaattttaa tcaaaaaatg ccgtcgggct tccaa 35
<210> 6
   <211> 52
   <212> DNA
   <213> Homo sapiens
<400> 6
   acatgactcg aggtcgacgg tatcgataag cttatcagtc gttctcatct gg 52
<210> 7
   <211> 73
   <212> DNA
   <213> Rattus norvegicus
<400> 7
   caaagaataa acacaaaaac aaaaagtttt tttaatttta atcaaaaaat gtctgaattc 60
   agcagcaaga agg 73
<210> 8
   <211> 71
   <212> DNA
   <213> Rattus norvegicus
<400> 8
   aagtttcttt gtctccgtcc cactcaactt tctgagaaca aatgatcgac aaataatagg 60
   tttaggtaag g 71
<210> 9
   <211> 7828
   <212> DNA
   <213> Homo sapiens
<400> 9
   atgccgtcgg gcttccaaca gataggctcc gaagatgggg aaccccctca gcagcgagtg 60
   actgggaccc tggtccttgc tgtgttctct gcggtgcttg gctccctgca gtttgggtac 120
   aacattgggg tcatcaatgc ccctcagaag gtgattgaac agagctacaa tgagacgtgg 180
   ctggggaggc aggggcctga gggacccagc tccatccctc caggcaccct caccaccctc 240
   tgggccctct ccgtggccat cttttccgtg ggcggcatga tttcctcctt cctcattggt 300
   atcatctctc agtggcttgg aaggaaaagg gccatgctgg tcaacaatgt cctggcggtg 360
   ctggggggca gcctcatggg cctggccaac gctgctgcct cctatgaaat gctcatcctt 420
   ggacgattcc tcattggcgc ctactcaggg ctgacatcag ggctggtgcc catgtacgtg 480
   ggggagattg ctcccactca cctgcggggc gccctgggga cgctcaacca actggccatt 540
   gttatcggca ttctgatcgc ccaggtgctg ggcttggagt ccctcctggg cactgccagc 600
   ctgtggccac tgctcctggg cctcacagtg ctacctgccc tcctgcagct ggtcctgctg 660
   cccttctgtc ccgagagccc ccgctacctc tacatcatcc agaatctcga ggggcctgcc 720
   agaaagagtc tgaagcgcct gacaggctgg gccgatgttt ctggagtgct ggctgagctg 780
   aaggatgaga agcggaagct ggagcgtgag cggccactgt ccctgctcca gctcctgggc 840
   agccgtaccc accggcagcc cctgatcatt gcggtcgtgc tgcagctgag ccagcagctc 900
   tctggcatca atgctgtttt ctattattcg accagcatct tcgagacagc aggggtaggc 960
   cagcctgcct atgccaccat aggagctggt gtggtcaaca cagtcttcac cttggtctcg 1020
   gtgttgttgg tggagcgggc ggggcgccgg acgctccatc tcctgggcct ggcgggcatg 1080
   tgtggctgtg ccatcctgat gactgtggct ctgctcctgc tggagcgagt tccagccatg 1140
   agctacgtct ccattgtggc catctttggc ttcgtggcat tttttgagat tggccctggc 1200
   cccattcctt ggttcatcgt ggccgagctc ttcagccagg gaccccgccc ggcagccatg 1260
   gctgtggctg gtttctccaa ctggacgagc aacttcatca ttggcatggg tttccagtat 1320
   gttgcggagg ctatggggcc ctacgtcttc cttctatttg cggtcctcct gctgggcttc 1380
   ttcatcttca ccttcttaag agtacctgaa actcgaggcc ggacgtttga ccagatctca 1440
   gctgccttcc accggacacc ctctctttta gagcaggagg tgaaacccag cacagaactt 1500
   gagtatttag ggccagatga gaacgactga taagcttatc gataccgtcg acctcgagtc 1560
   atgtaattag ttatgtcacg cttacattca cgccctcccc ccacatccgc tctaaccgaa 1620
   aaggaaggag ttagacaacc tgaagtctag gtccctattt atttttttat agttatgtta 1680
   gtattaagaa cgttatttat atttcaaatt tttctttttt ttctgtacag acgcgtgtac 1740
   gcatgtaaca ttatactgaa aaccttgctt gagaaggttt tgggacgctc gaaggcttta 1800
   atttgcggcc ggtacccaat tcgccctata gtgagtcgta ttacgcgcgc tcactggccg 1860
   tcgttttaca acgtcgtgac tgggaaaacc ctggcgttac ccaacttaat cgccttgcag 1920
   cacatccccc tttcgccagc tggcgtaata gcgaagaggc ccgcaccgat cgcccttccc 1980
   aacagttgcg cagcctgaat ggcgaatggc gcgacgcgcc ctgtagcggc gcattaagcg 2040
   cggcgggtgt ggtggttacg cgcagcgtga ccgctacact tgccagcgcc ctagcgcccg 2100
   ctcctttcgc tttcttccct tcctttctcg ccacgttcgc cggctttccc cgtcaagctc 2160
   taaatcgggg gctcccttta gggttccgat ttagtgcttt acggcacctc gaccccaaaa 2220
   aacttgatta gggtgatggt tcacgtagtg ggccatcgcc ctgatagacg gtttttcgcc 2280
   ctttgacgtt ggagtccacg ttctttaata gtggactctt gttccaaact ggaacaacac 2340
   tcaaccctat ctcggtctat tcttttgatt tataagggat tttgccgatt tcggcctatt 2400
   ggttaaaaaa tgagctgatt taacaaaaat ttaacgcgaa ttttaacaaa atattaacgt 2460
   ttacaatttc ctgatgcggt attttctcct tacgcatctg tgcggtattt cacaccgcat 2520
   agggtaataa ctgatataat taaattgaag ctctaatttg tgagtttagt atacatgcat 2580
   ttacttataa tacagttttt tagttttgct ggccgcatct tctcaaatat gcttcccagc 2640
   ctgcttttct gtaacgttca ccctctacct tagcatccct tccctttgca aatagtcctc 2700
   ttccaacaat aataatgtca gatcctgtag agaccacatc atccacggtt ctatactgtt 2760
   gacccaatgc gtctcccttg tcatctaaac ccacaccggg tgtcataatc aaccaatcgt 2820
   aaccttcatc tcttccaccc atgtctcttt gagcaataaa gccgataaca aaatctttgt 2880
   cgctcttcgc aatgtcaaca gtacccttag tatattctcc agtagatagg gagcccttgc 2940
   atgacaattc tgctaacatc aaaaggcctc taggttcctt tgttacttct tctgccgcct 3000
   gcttcaaacc gctaacaata cctgggccca ccacaccgtg tgcattcgta atgtctgccc 3060
   attctgctat tctgtataca cccgcagagt actgcaattt gactgtatta ccaatgtcag 3120
   caaattttct gtcttcgaag agtaaaaaat tgtacttggc ggataatgcc tttagcggct 3180
   taactgtgcc ctccatggaa aaatcagtca agatatccac atgtgttttt agtaaacaaa 3240
   ttttgggacc taatgcttca actaactcca gtaattcctt ggtggtacga acatccaatg 3300
   aagcacacaa gtttgtttgc ttttcgtgca tgatattaaa tagcttggca gcaacaggac 3360
   taggatgagt agcagcacgt tccttatatg tagctttcga catgatttat cttcgtttcc 3420
   tgcaggtttt tgttctgtgc agttgggtta agaatactgg gcaatttcat gtttcttcaa 3480
   cactacatat gcgtatatat accaatctaa gtctgtgctc cttccttcgt tcttccttct 3540
   gttcggagat taccgaatca aaaaaatttc aaagaaaccg aaatcaaaaa aaagaataaa 3600
   aaaaaaatga tgaattgaat tgaaaagctg tggtatggtg cactctcagt acaatctgct 3660
   ctgatgccgc atagttaagc cagccccgac acccgccaac acccgctgac gcgccctgac 3720
   gggcttgtct gctcccggca tccgcttaca gacaagctgt gaccgtctcc gggagctgca 3780
   tgtgtcagag gttttcaccg tcatcaccga aacgcgcgag acgaaagggc ctcgtgatac 3840
   gcctattttt ataggttaat gtcatgataa taatggtttc ttagtatgat ccaatatcaa 3900
   aggaaatgat agcattgaag gatgagacta atccaattga ggagtggcag catatagaac 3960
   agctaaaggg tagtgctgaa ggaagcatac gataccccgc atggaatggg ataatatcac 4020
   aggaggtact agactacctt tcatcctaca taaatagacg catataagta cgcatttaag 4080
   cataaacacg cactatgccg ttcttctcat gtatatatat atacaggcaa cacgcagata 4140
   taggtgcgac gtgaacagtg agctgtatgt gcgcagctcg cgttgcattt tcggaagcgc 4200
   tcgttttcgg aaacgctttg aagttcctat tccgaagttc ctattctcta gaaagtatag 4260
   gaacttcaga gcgcttttga aaaccaaaag cgctctgaag acgcactttc aaaaaaccaa 4320
   aaacgcaccg gactgtaacg agctactaaa atattgcgaa taccgcttcc acaaacattg 4380
   ctcaaaagta tctctttgct atatatctct gtgctatatc cctatataac ctacccatcc 4440
   acctttcgct ccttgaactt gcatctaaac tcgacctcta cattttttat gtttatctct 4500
   agtattactc tttagacaaa aaaattgtag taagaactat tcatagagtg aatcgaaaac 4560
   aatacgaaaa tgtaaacatt tcctatacgt agtatataga gacaaaatag aagaaaccgt 4620
   tcataatttt ctgaccaatg aagaatcatc aacgctatca ctttctgttc acaaagtatg 4680
   cgcaatccac atcggtatag aatataatcg ggatgccttt tatcttgaaa aaatgcaccc 4740
   gcagcttcgc tagtaatcag taaacgcggg aagtggagtc aggctttttt tatggaagag 4800
   aaaatagaca ccaaagtagc cttcttctaa ccttaacgga cctacagtgc aaaaagttat 4860
   caagagactg cattatagag cgcacaaagg agaaaaaaag taatctaaga tgctttgtta 4920
   gaaaaatagc gctctcggga tgcatttttg tagaacaaaa aagaagtata gattctttgt 4980
   tggtaaaata gcgctctcgc gttgcatttc tgttctgtaa aaatgcagct cagattcttt 5040
   gtttgaaaaa ttagcgctct cgcgttgcat tttgttttta caaaaatgaa gcacagattc 5100
   ttcgttggta aaatagcgct ttcgcgttgc atttctgttc tgtaaaaatg cagctcagat 5160
   tctttgtttg aaaaattagc gctctcgcgt tgcatttttg ttctacaaaa tgaagcacag 5220
   atgcttcgtt caggtggcac ttttcgggga aatgtgcgcg gaacccctat ttgtttattt 5280
   ttctaaatac attcaaatat gtatccgctc atgagacaat aaccctgata aatgcttcaa 5340
   taatattgaa aaaggaagag tatgagtatt caacatttcc gtgtcgccct tattcccttt 5400
   tttgcggcat tttgccttcc tgtttttgct cacccagaaa cgctggtgaa agtaaaagat 5460
   gctgaagatc agttgggtgc acgagtgggt tacatcgaac tggatctcaa cagcggtaag 5520
   atccttgaga gttttcgccc cgaagaacgt tttccaatga tgagcacttt taaagttctg 5580
   ctatgtggcg cggtattatc ccgtattgac gccgggcaag agcaactcgg tcgccgcata 5640
   cactattctc agaatgactt ggttgagtac tcaccagtca cagaaaagca tcttacggat 5700
   ggcatgacag taagagaatt atgcagtgct gccataacca tgagtgataa cactgcggcc 5760
   aacttacttc tgacaacgat cggaggaccg aaggagctaa ccgctttttt gcacaacatg 5820
   ggggatcatg taactcgcct tgatcgttgg gaaccggagc tgaatgaagc cataccaaac 5880
   gacgagcgtg acaccacgat gcctgtagca atggcaacaa cgttgcgcaa actattaact 5940
   ggcgaactac ttactctagc ttcccggcaa caattaatag actggatgga ggcggataaa 6000
   gttgcaggac cacttctgcg ctcggccctt ccggctggct ggtttattgc tgataaatct 6060
   ggagccggtg agcgtgggtc tcgcggtatc attgcagcac tggggccaga tggtaagccc 6120
   tcccgtatcg tagttatcta cacgacgggg agtcaggcaa ctatggatga acgaaataga 6180
   cagatcgctg agataggtgc ctcactgatt aagcattggt aactgtcaga ccaagtttac 6240
   tcatatatac tttagattga tttaaaactt catttttaat ttaaaaggat ctaggtgaag 6300
   atcctttttg ataatctcat gaccaaaatc ccttaacgtg agttttcgtt ccactgagcg 6360
   tcagaccccg tagaaaagat caaaggatct tcttgagatc ctttttttct gcgcgtaatc 6420
   tgctgcttgc aaacaaaaaa accaccgcta ccagcggtgg tttgtttgcc ggatcaagag 6480
   ctaccaactc tttttccgaa ggtaactggc ttcagcagag cgcagatacc aaatactgtc 6540
   cttctagtgt agccgtagtt aggccaccac ttcaagaact ctgtagcacc gcctacatac 6600
   ctcgctctgc taatcctgtt accagtggct gctgccagtg gcgataagtc gtgtcttacc 6660
   gggttggact caagacgata gttaccggat aaggcgcagc ggtcgggctg aacggggggt 6720
   tcgtgcacac agcccagctt ggagcgaacg acctacaccg aactgagata cctacagcgt 6780
   gagctatgag aaagcgccac gcttcccgaa gggagaaagg cggacaggta tccggtaagc 6840
   ggcagggtcg gaacaggaga gcgcacgagg gagcttccag ggggaaacgc ctggtatctt 6900
   tatagtcctg tcgggtttcg ccacctctga cttgagcgtc gatttttgtg atgctcgtca 6960
   ggggggcgga gcctatggaa aaacgccagc aacgcggcct ttttacggtt cctggccttt 7020
   tgctggcctt ttgctcacat gttctttcct gcgttatccc ctgattctgt ggataaccgt 7080
   attaccgcct ttgagtgagc tgataccgct cgccgcagcc gaacgaccga gcgcagcgag 7140
   tcagtgagcg aggaagcgga agagcgccca atacgcaaac cgcctctccc cgcgcgttgg 7200
   ccgattcatt aatgcagctg gcacgacagg tttcccgact ggaaagcggg cagtgagcgc 7260
   aacgcaatta atgtgagtta cctcactcat taggcacccc aggctttaca ctttatgctt 7320
   ccggctccta tgttgtgtgg aattgtgagc ggataacaat ttcacacagg aaacagctat 7380
   gaccatgatt acgccaagcg cgcaattaac cctcactaaa gggaacaaaa gctggagctc 7440
   gtaggaacaa tttcgggccc ctgcgtgttc ttctgaggtt catcttttac atttgcttct 7500
   gctggataat tttcagaggc aacaaggaaa aattagatgg caaaaagtcg tctttcaagg 7560
   aaaaatcccc accatctttc gagatcccct gtaacttatt ggcaactgaa agaatgaaaa 7620
   ggaggaaaat acaaaatata ctagaactga aaaaaaaaaa gtataaatag agacgatata 7680
   tgccaatact tcacaatgtt cgaatctatt cttcatttgc agctattgta aaataataaa 7740
   acatcaagaa caaacaagct caacttgtct tttctaagaa caaagaataa acacaaaaac 7800
   aaaaagtttt tttaatttta atcaaaaa 7828
<210> 10
   <211> 2386
   <212> DNA
   <213> Rattus norvegicus
<400> 10
   tcgactctag aggatcccct taagctaatc cttatgaatc cggagaaaag cggggtcttt 60
   taactcaata aaattttccg aaatcctttt tcctacgcgt tttcttcggg aactagatag 120
   gtggctcttc cacctgtttt tccatcattt tagtttttcg caagccatgc gtgccttttc 180
   gtttttgcga tggcgaacga gggctggaaa aattaacggt acgccgccta acgatagtaa 240
   taggccacgc aactggcgtg gacgacaaca ataagtcgcc cattttttat gttttcaaaa 300
   cctagcaacc cccaccaaac ttgtcatcgt tcccggattc acaaatgata taaaaagcga 360
   ttacaattct acattctaac cagatttgag atttcctctt tctcaattcc tcttatatta 420
   gattataaga acaacaaatt aaattacaaa aagacttata aagcaacata atgtctgaat 480
   tccagcagat cggctctgaa gatggggaac cccctcagca gcgagtgact gggacactgg 540
   tccttgctgt attctcagct gtgcttggct cccttcagtt tggctataac attggagtca 600
   tcaacgcccc acagaaagtg attgaacaga gctacaatgc aacttggctg ggtaggcagg 660
   gtcctggggg accggactcc atcccacaag gcaccctcac taccctttgg gctctctccg 720
   tggccatctt ctctgtgggt ggcatgattt cctcctttct cattggcatc atttctcaat 780
   ggttgggaag gaaaagggct atgctggcca acaatgtctt ggctgtgctg gggggcgccc 840
   tcatgggcct agccaatgcc gcggcctcct atgagatact cattctcgga cggttcctca 900
   ttggcgccta ctcagggcta acatcagggt tggtgcctat gtatgtggga gaaatcgccc 960
   ccactcatct tcggggtgcc ttgggaacac tcaaccaatt ggccatcgtc attggcattc 1020
   tggttgccca ggtgttgggt ttggagtcta tgctgggcac agctaccctg tggccattgc 1080
   ttctggctat cacagtactc cctgctctcc tgcagctgct tctgttgccc ttctgtcctg 1140
   agagcccccg atacctctac atcatccgga acctggaggg gcctgcccga aagagtctaa 1200
   agcgcctgac aggctgggct gatgtgtctg atgcactggc tgagctgaag gatgagaaac 1260
   ggaagttgga aagagagcgt ccactgtcct tgctgcagct cctgggcagc cgcacccacc 1320
   ggcagcctct gattattgca gtggtgctgc agctgagcca gcagctctca ggcatcaatg 1380
   ctgttttcta ctattcaacc agcatctttg agttagctgg ggtggaacag ccagcctacg 1440
   ccaccatagg agctggtgtg gtcaataccg tcttcacgtt ggtctcggtg ctcttagtag 1500
   agcgagctgg gcgacggaca ctccatctcc tgggcctggc aggcatgtgt ggctgtgcca 1560
   tcttgatgac ggtggctctg ctgctgctgg agcgggttcc atccatgagt tatgtgtcca 1620
   tcgtggccat atttggcttt gtggccttct ttgagattgg tcctggcccc atcccctggt 1680
   tcattgtggc cgagctcttc agccagggcc cccgcccagc agccatggct gtagctggtt 1740
   tctccaactg gacctgtaac ttcatcgttg gcatgggttt ccagtatgtt gcggatgcta 1800
   tgggtcccta cgtcttcctt ctatttgccg tcctcctgct tggcttcttc atcttcacct 1860
   tcctaagagt gcctgaaacc agaggccgga catttgacca gatctcggcc accttccgac 1920
   ggacaccttc tctcttagag caggaggtga aacccagtac agaacttgaa tacttagggc 1980
   cagatgagaa tgactaatcg atttgaagtg agacgctcca tcatctctct taatttttca 2040
   tgactgacgt tttttcttca ttttaattat catagtattt gtttgaaaaa aaaaaaaaaa 2100
   aatttccctt atcaatgata tccttacgat tatataaatt ccttacctaa acctattatt 2160
   tgtgtacata tatcagagta ttattacata tataaccttt ttctctaaaa caggaaaaaa 2220
   aaaagaaaac gataacatgc tctgccatcc tttgttcacc gagcaaaatt aaaaacgcaa 2280
   aatgaattgt ccctatgaaa ttattaaagg accacatcac cagacttatc tctggggggt 2340
   cctctagaaa ataagtcagg tacttgcctg gactttcttc cagttg                2386
<210> 11
   <211> 7777
   <212> DNA
   <213> Homo sapiens
<400> 11
   atggagccca gcagcaagaa gctgacgggt cgcctcatgc tggctgtggg aggagcagtg 60
   cttggctccc tgcagtttgg ctacaacact ggagtcatca atgcccccca gaaggtgatc 120
   gaggagttct acaaccagac atgggtccac cgctatgggg agagcatcct gcccaccacg 180
   ctcaccacgc tctggtccct ctcagtggcc atcttttctg ttgggggcat gattggctcc 240
   ttctctgtgg gccttttcgt taaccgcttt ggccggcgga attcaatgct gatgatgaac 300
   ctgctggcct tcgtgtccgc cgtgctcatg ggcttctcga aactgggcaa gtcctttgag 360
   atgctgatcc tgggccgctt catcatcggt gtgtactgcg gcctgaccac aggcttcgtg 420
   cccatgtatg tgggtgaagt gtcacccaca gcctttcgtg gggccctggg caccctgcac 480
   cagctgggca tcgtcgtcgg catcctcatc gcccaggtgt tcggcctgga ctccatcatg 540
   ggcaacaagg acctgtggcc cctgctgctg agcatcatct tcatcccggc cctgctgcag 600
   tgcatcgtgc tgcccttctg ccccgagagt ccccgcttcc tgctcatcaa ccgcaacgag 660
   gagaaccggg ccaagagtgt gctaaagaag ctgcgcggga cagctgacgt gacccatgac 720
   ctgcaggaga tgaaggaaga gagtcggcag atgatgcggg agaagaaggt caccatcctg 780
   gagctgttcc gctcccccgc ctaccgccag cccatcctca tcgctgtggt gctgcagctg 840
   tcccagcagc tgtctggcat caacgctgtc ttctattact ccacgagcat cttcgagaag 900
   gcgggggtgc agcagcctgt gtatgccacc attggctccg gtatcgtcaa cacggccttc 960
   actgtcgtgt cgctgtttgt ggtggagcga gcaggccggc ggaccctgca cctcataggc 1020
   ctcgctggca tggcgggttg tgccatactc atgaccatcg cgctagcact gctggagcag 1080
   ctaccctgga tgtcctatct gagcatcgtg gccatctttg gctttgtggc cttctttgaa 1140
   gtgggtcctg gccccatccc atggttcatc gtggctgaac tcttcagcca gggtccacgt 1200
   ccagctgcca ttgccgttgc aggcttctcc aactggacct caaatttcat tgtgggcatg 1260
   tgcttccagt atgtggagca actgtgtggt ccctacgtct tcatcatctt cactgtgctc 1320
   ctggttctgt tcttcatctt cacctacttc aaagttcctg agactaaagg ccggaccttc 1380
   gatgagatcg cttccggctt ccggcagggg ggagccagcc aaagtgataa gacacccgag 1440
   gagctgttcc atcccctggg ggctgattcc caagtgtgat aagcttatcg ataccgtcga 1500
   cctcgagtca tgtaattagt tatgtcacgc ttacattcac gccctccccc cacatccgct 1560
   ctaaccgaaa aggaaggagt tagacaacct gaagtctagg tccctattta tttttttata 1620
   gttatgttag tattaagaac gttatttata tttcaaattt ttcttttttt tctgtacaga 1680
   cgcgtgtacg catgtaacat tatactgaaa accttgcttg agaaggtttt gggacgctcg 1740
   aaggctttaa tttgcggccg gtacccaatt cgccctatag tgagtcgtat tacgcgcgct 1800
   cactggccgt cgttttacaa cgtcgtgact gggaaaaccc tggcgttacc caacttaatc 1860
   gccttgcagc acatccccct ttcgccagct ggcgtaatag cgaagaggcc cgcaccgatc 1920
   gcccttccca acagttgcgc agcctgaatg gcgaatggcg cgacgcgccc tgtagcggcg 1980
   cattaagcgc ggcgggtgtg gtggttacgc gcagcgtgac cgctacactt gccagcgccc 2040
   tagcgcccgc tcctttcgct ttcttccctt cctttctcgc cacgttcgcc ggctttcccc 2100
   gtcaagctct aaatcggggg ctccctttag ggttccgatt tagtgcttta cggcacctcg 2160
   accccaaaaa acttgattag ggtgatggtt cacgtagtgg gccatcgccc tgatagacgg 2220
   tttttcgccc tttgacgttg gagtccacgt tctttaatag tggactcttg ttccaaactg 2280
   gaacaacact caaccctatc tcggtctatt cttttgattt ataagggatt ttgccgattt 2340
   cggcctattg gttaaaaaat gagctgattt aacaaaaatt taacgcgaat tttaacaaaa 2400
   tattaacgtt tacaatttcc tgatgcggta ttttctcctt acgcatctgt gcggtatttc 2460
   acaccgcata gggtaataac tgatataatt aaattgaagc tctaatttgt gagtttagta 2520
   tacatgcatt tacttataat acagtttttt agttttgctg gccgcatctt ctcaaatatg 2580
   cttcccagcc tgcttttctg taacgttcac cctctacctt agcatccctt ccctttgcaa 2640
   atagtcctct tccaacaata ataatgtcag atcctgtaga gaccacatca tccacggttc 2700
   tatactgttg acccaatgcg tctcccttgt catctaaacc cacaccgggt gtcataatca 2760
   accaatcgta accttcatct cttccaccca tgtctctttg agcaataaag ccgataacaa 2820
   aatctttgtc gctcttcgca atgtcaacag tacccttagt atattctcca gtagataggg 2880
   agcccttgca tgacaattct gctaacatca aaaggcctct aggttccttt gttacttctt 2940
   ctgccgcctg cttcaaaccg ctaacaatac ctgggcccac cacaccgtgt gcattcgtaa 3000
   tgtctgccca ttctgctatt ctgtatacac ccgcagagta ctgcaatttg actgtattac 3060
   caatgtcagc aaattttctg tcttcgaaga gtaaaaaatt gtacttggcg gataatgcct 3120
   ttagcggctt aactgtgccc tccatggaaa aatcagtcaa gatatccaca tgtgttttta 3180
   gtaaacaaat tttgggacct aatgcttcaa ctaactccag taattccttg gtggtacgaa 3240
   catccaatga agcacacaag tttgtttgct tttcgtgcat gatattaaat agcttggcag 3300
   caacaggact aggatgagta gcagcacgtt ccttatatgt agctttcgac atgatttatc 3360
   ttcgtttcct gcaggttttt gttctgtgca gttgggttaa gaatactggg caatttcatg 3420
   tttcttcaac actacatatg cgtatatata ccaatctaag tctgtgctcc ttccttcgtt 3480
   cttccttctg ttcggagatt accgaatcaa aaaaatttca aagaaaccga aatcaaaaaa 3540
   aagaataaaa aaaaaatgat gaattgaatt gaaaagctgt ggtatggtgc actctcagta 3600
   caatctgctc tgatgccgca tagttaagcc agccccgaca cccgccaaca cccgctgacg 3660
   cgccctgacg ggcttgtctg ctcccggcat ccgcttacag acaagctgtg accgtctccg 3720
   ggagctgcat gtgtcagagg ttttcaccgt catcaccgaa acgcgcgaga cgaaagggcc 3780
   tcgtgatacg cctattttta taggttaatg tcatgataat aatggtttct tagtatgatc 3840
   caatatcaaa ggaaatgata gcattgaagg atgagactaa tccaattgag gagtggcagc 3900
   atatagaaca gctaaagggt agtgctgaag gaagcatacg ataccccgca tggaatggga 3960
   taatatcaca ggaggtacta gactaccttt catcctacat aaatagacgc atataagtac 4020
   gcatttaagc ataaacacgc actatgccgt tcttctcatg tatatatata tacaggcaac 4080
   acgcagatat aggtgcgacg tgaacagtga gctgtatgtg cgcagctcgc gttgcatttt 4140
   cggaagcgct cgttttcgga aacgctttga agttcctatt ccgaagttcc tattctctag 4200
   aaagtatagg aacttcagag cgcttttgaa aaccaaaagc gctctgaaga cgcactttca 4260
   aaaaaccaaa aacgcaccgg actgtaacga gctactaaaa tattgcgaat accgcttcca 4320
   caaacattgc tcaaaagtat ctctttgcta tatatctctg tgctatatcc ctatataacc 4380
   tacccatcca cctttcgctc cttgaacttg catctaaact cgacctctac attttttatg 4440
   tttatctcta gtattactct ttagacaaaa aaattgtagt aagaactatt catagagtga 4500
   atcgaaaaca atacgaaaat gtaaacattt cctatacgta gtatatagag acaaaataga 4560
   agaaaccgtt cataattttc tgaccaatga agaatcatca acgctatcac tttctgttca 4620
   caaagtatgc gcaatccaca tcggtataga atataatcgg ggatgccttt atcttgaaaa 4680
   aatgcacccg cagcttcgct agtaatcagt aaacgcggga agtggagtca ggcttttttt 4740
   atggaagaga aaatagacac caaagtagcc ttcttctaac cttaacggac ctacagtgca 4800
   aaaagttatc aagagactgc attatagagc gcacaaagga gaaaaaaagt aatctaagat 4860
   gctttgttag aaaaatagcg ctctcgggat gcatttttgt agaacaaaaa agaagtatag 4920
   attctttgtt ggtaaaatag cgctctcgcg ttgcatttct gttctgtaaa aatgcagctc 4980
   agattctttg tttgaaaaat tagcgctctc gcgttgcatt tttgttttac aaaaatgaag 5040
   cacagattct tcgttggtaa aatagcgctt tcgcgttgca tttctgttct gtaaaaatgc 5100
   agctcagatt ctttgtttga aaaattagcg ctctcgcgtt gcatttttgt tctacaaaat 5160
   gaagcacaga tgcttcgttc aggtggcact tttcggggaa atgtgcgcgg aacccctatt 5220
   tgtttatttt tctaaataca ttcaaatatg tatccgctca tgagacaata accctgataa 5280
   atgcttcaat aatattgaaa aaggaagagt atgagtattc aacatttccg tgtcgccctt 5340
   attccctttt ttgcggcatt ttgccttcct gtttttgctc acccagaaac gctggtgaaa 5400
   gtaaaagatg ctgaagatca gttgggtgca cgagtgggtt acatcgaact ggatctcaac 5460
   agcggtaaga tccttgagag ttttcgcccc gaagaacgtt ttccaatgat gagcactttt 5520
   aaagttctgc tatgtggcgc ggtattatcc cgtattgacg ccgggcaaga gcaactcggt 5580
   cgccgcatac actattctca gaatgacttg gttgagtact caccagtcac agaaaagcat 5640
   cttacggatg gcatgacagt aagagaatta tgcagtgctg ccataaccat gagtgataac 5700
   actgcggcca acttacttct gacaacgatc ggaggaccga aggagctaac cgcttttttg 5760
   cacaacatgg gggatcatgt aactcgcctt gatcgttggg aaccggagct gaatgaagcc 5820
   ataccaaacg acgagcgtga caccacgatg cctgtagcaa tggcaacaac gttgcgcaaa 5880
   ctattaactg gcgaactact tactctagct tcccggcaac aattaataga ctggatggag 5940
   gcggataaag ttgcaggacc acttctgcgc tcggcccttc cggctggctg gtttattgct 6000
   gataaatctg gagccggtga gcgtgggtct cgcggtatca ttgcagcact ggggccagat 6060
   ggtaagccct cccgtatcgt agttatctac acgacgggga gtcaggcaac tatggatgaa 6120
   cgaaatagac agatcgctga gataggtgcc tcactgatta agcattggta actgtcagac 6180
   caagtttact catatatact ttagattgat ttaaaacttc atttttaatt taaaaggatc 6240
   taggtgaaga tcctttttga taatctcatg accaaaatcc cttaacgtga gttttcgttc 6300
   cactgagcgt cagaccccgt agaaaagatc aaaggatctt cttgagatcc tttttttctg 6360
   cgcgtaatct gctgcttgca aacaaaaaaa ccaccgctac cagcggtggt ttgtttgccg 6420
   gatcaagagc taccaactct ttttccgaag gtaactggct tcagcagagc gcagatacca 6480
   aatactgtcc ttctagtgta gccgtagtta ggccaccact tcaagaactc tgtagcaccg 6540
   cctacatacc tcgctctgct aatcctgtta ccagtggctg ctgccagtgg cgataagtcg 6600
   tgtcttaccg ggttggactc aagacgatag ttaccggata aggcgcagcg gtcgggctga 6660
   acggggggtt cgtgcacaca gcccagcttg gagcgaacga cctacaccga actgagatac 6720
   ctacagcgtg agctatgaga aagcgccacg cttcccgaag ggagaaaggc ggacaggtat 6780
   ccggtaagcg gcagggtcgg aacaggagag cgcacgaggg agcttccagg gggaaacgcc 6840
   tggtatcttt atagtcctgt cgggtttcgc cacctctgac ttgagcgtcg atttttgtga 6900
   tgctcgtcag gggggcggag cctatggaaa aacgccagca acgcggcctt tttacggttc 6960
   ctggcctttt gctggccttt tgctcacatg ttctttcctg cgttatcccc tgattctgtg 7020
   gataaccgta ttaccgcctt tgagtgagct gataccgctc gccgcagccg aacgaccgag 7080
   cgcagcgagt cagtgagcga ggaagcggaa gagcgcccaa tacgcaaacc gcctctcccc 7140
   gcgcgttggc cgattcatta atgcagctgg cacgacaggt ttcccgactg gaaagcgggc 7200
   agtgagcgca acgcaattaa tgtgagttac ctcactcatt aggcacccca ggctttacac 7260
   tttatgcttc cggctcctat gttgtgtgga attgtgagcg gataacaatt tcacacagga 7320
   aacagctatg accatgatta cgccaagcgc gcaattaacc ctcactaaag ggaacaaaag 7380
   ctggagctcg taggaacaat ttcgggcccc tgcgtgttct tctgaggttc atcttttaca 7440
   tttgcttctg ctggataatt ttcagaggca acaaggaaaa attagatggc aaaaagtcgt 7500
   ctttcaagga aaaatcccca ccatctttcg agatcccctg taacttattg gcaactgaaa 7560
   gaatgaaaag gaggaaaata caaaatatac tagaactgaa aaaaaaaaag tataaataga 7620
   gacgatatat gccaatactt cacaatgttc gaatctattc ttcatttgca gctattgtaa 7680
   aataataaaa catcaagaac aaacaagctc aacttgtctt ttctaagaac aaagaataaa 7740
   cacaaaaaca aaaagttttt ttaattttaa tcaaaaa                          7777
<210> 12
   <211> 2338
   <212> DNA
   <213> Rattus norvegicus
<400> 12
   tcgactctag aggatcccct taagctaatc cttatgaatc cggagaaaag cggggtcttt 60
   taactcaata aaattttccg aaatcctttt tcctacgcgt tttcttcggg aactagatag 120
   gtggctcttc cacctgtttt tccatcattt tagtttttcg caagccatgc gtgccttttc 180
   gtttttgcga tggcgaacga gggctggaaa aattaacggt acgccgccta acgatagtaa 240
   taggccacgc aactggcgtg gacgacaaca ataagtcgcc cattttttat gttttcaaaa 300
   cctagcaacc cccaccaaac ttgtcatcgt tcccggattc acaaatgata taaaaagcga 360
   ttacaattct acattctaac cagatttgag atttcctctt tctcaattcc tcttatatta 420
   gattataaga acaacaaatt aaattacaaa aagacttata aagcaacata atgtctgaat 480
   tcagcaagaa ggtgacgggc cgccttatgt tggccgtggg aggggcagtg ctcggatccc 540
   tgcagttcgg ctataacacc ggtgtcatca acgcccccca gaaggtaatt gaggagttct 600
   acaatcaaac atggaaccac cgctatggag agtccatccc atccaccaca ctcaccacac 660
   tctggtctct ctccgtggcc atcttctctg tcgggggcat gattggttcc ttctctgtgg 720
   gcctctttgt taatcgcttt ggcaggcgga actccatgct gatgatgaac ctgttggcct 780
   ttgtgtctgc cgtgcttatg ggtttctcca aactgggcaa gtcctttgag atgctgatcc 840
   tgggccgctt catcattgga gtgtactgtg gcctgaccac cggctttgtg cccatgtatg 900
   tgggggaggt gtcacccaca gctcttcgtg gagccctggg caccctgcac cagctgggca 960
   tcgtcgttgg gatccttatt gcccaggtgt tcggcttaga ctccatcatg ggcaatgcag 1020
   acttgtggcc tctactgctc agtgtcatct tcatcccagc cctgctacag tgtatcctgt 1080
   tgcccttctg ccctgagagc ccccgcttcc tgctcatcaa tcgtaacgag gagaaccggg 1140
   ccaagagtgt gctgaaaaag cttcgaggga cagccgatgt gacccgagac ctgcaggaga 1200
   tgaaagaaga gggtcggcag atgatgcggg agaagaaggt caccatcttg gagctgttcc 1260
   gctcacccgc ctaccgccag cccatcctca tcgccgtggt gctgcagctg tcccagcagc 1320
   tgtcgggcat caatgctgtg ttctactact caacgagcat cttcgagaag gcaggtgtgc 1380
   agcagcctgt gtatgccacc atcggctcgg gtatcgtcaa cacggccttc actgtggtgt 1440
   cgctgttcgt cgtggagcga gctggccgtc ggaccctgca tctcattggt ctggctggca 1500
   tggcgggctg tgctgtgctc atgaccatcg ccctggccct gctggagcag ctgccctgga 1560
   tgtcctatct gagtatcgtg gccatctttg gctttgtggc cttctttgaa gtaggccctg 1620
   gtcctattcc atggttcatt gtggccgagc tgttcagcca ggggccccga cctgctgctg 1680
   ttgctgtggc tggcttctct aactggacct caaacttcat cgtgggcatg tgcttccaat 1740
   atgtggagca actgtgtggc ccctacgtct tcatcatctt cacggtgctg ctggtactct 1800
   tcttcatctt cacctacttc aaagttcctg agaccaaagg ccggaccttc gatgagatcg 1860
   cttccggctt ccggcagggg ggtgccagcc agagcgacaa gacacctgag gagctcttcc 1920
   accctctggg ggctgactcc caagtgtaat cgatttgaag tgagacgctc catcatctct 1980
   cttaattttt catgactgac gttttttctt cattttaatt atcatagtat ttgtttgaaa 2040
   aaaaaaaaaa aaaatttccc ttatcaatga tatccttacg attatataaa ttccttacct 2100
   aaacctatta tttgtgtaca tatatcagag tattattaca tatataacct ttttctctaa 2160
   aacaggaaaa aaaaaagaaa acgataacat gctctgccat cctttgttca ccgagcaaaa 2220
   ttaaaaacgc aaaatgaatt gtccctatga aattattaaa ggaccacatc accagactta 2280
   tctctggggg gtcctctaga aaataagtca ggtacttgcc tggactttct tccagttg   2338
<210> 13
   <211> 2338
   <212> DNA
   <213> Rattus norvegicus
<400> 13
   tcgactctag aggatcccct taagctaatc cttatgaatc cggagaaaag cggggtcttt 60
   taactcaata aaattttccg aaatcctttt tcctacgcgt tttcttcggg aactagatag 120
   gtggctcttc cacctgtttt tccatcattt tagtttttcg caagccatgc gtgccttttc 180
   gtttttgcga tggcgaacga gggctggaaa aattaacggt acgccgccta acgatagtaa 240
   taggccacgc aactggcgtg gacgacaaca ataagtcgcc cattttttat gttttcaaaa 300
   cctagcaacc cccaccaaac ttgtcatcgt tcccggattc acaaatgata taaaaagcga 360
   ttacaattct acattctaac cagatttgag atttcctctt tctcaattcc tcttatatta 420
   gattataaga acaacaaatt aaattacaaa aagacttata aagcaacata atgtctgaat 480
   tcagcaagaa ggtgacgggc cgccttatgt tggccgtggg aggggcagtg ctcggatccc 540
   tgcagttcgg ctataacacc ggtgtcatca acgcccccca gaaggtaatt gaggagttct 600
   acaatcaaac atggaaccac cgctatggag agtccatccc atccaccaca ctcaccacac 660
   tctggtctct ctccatggcc atcttctctg tcgggggcat gattggttcc ttctctgtgg 720
   gcctctttgt taatcgcttt ggcaggcgga actccatgct gatgatgaac ctgttggcct 780
   ttgtgtctgc cgtgcttatg ggtttctcca aactgggcaa gtcctttgag atgctgatcc 840
   tgggccgctt catcattgga gtgtactgtg gcctgaccac cggctttgtg cccatgtatg 900
   tgggggaggt gtcacccaca gctcttcgtg gagccctggg caccctgcac cagctgggca 960
   tcgtcgttgg gatccttatt gcccaggtgt tcggcttaga ctccatcatg ggcaatgcag 1020
   acttgtggcc tctactgctc agtgtcatct tcatcccagc cctgctacag tgtatcctgt 1080
   tgcccttctg ccctgagagc ccccgcttcc tgctcatcaa tcgtaacgag gagaaccggg 1140
   ccaagagtgt gctgaaaaag cttcgaggga cagccgatgt gacccgagac ctgcaggaga 1200
   tgaaagaaga gggtcggcag atgatgcggg agaagaaggt caccatcttg gagctgttcc 1260
   gctcacccgc ctaccgccag cccatcctca tcgccgtggt gctgcagctg tcccagcagc 1320
   tgtcgggcat caatgctgtg ttctactact caacgagcat cttcgagaag gcaggtgtgc 1380
   agcagcctgt gtatgccacc atcggctcgg gtatcgtcaa cacggccttc actgtggtgt 1440
   cgctgttcgt cgtggagcga gctggccgtc ggaccctgca tctcattggt ctggctggca 1500
   tggcgggctg tgctgtgctc atgaccatcg ccctggccct gctggagcag ctgccctgga 1560
   tgtcctatct gagtatcgtg gccatctttg gctttgtggc cttctttgaa gtaggccctg 1620
   gtcctattcc atggttcatt gtggccgagc tgttcagcca ggggccccga cctgctgctg 1680
   ttgctgtggc tggcttctct aactggacct caaacttcat cgtgggcatg tgcttccaat 1740
   atgtggagca actgtgtggc ccctacgtct tcatcatctt cacggtgctg ctggtactct 1800
   tcttcatctt cacctacttc aaagttcctg agaccaaagg ccggaccttc gatgagatcg 1860
   cttccggctt ccggcagggg ggtgccagcc agagcgacaa gacacctgag gagctcttcc 1920
   accctctggg ggctgactcc caagtgtaat cgatttgaag tgagacgctc catcatctct 1980
   cttaattttt catgactgac gttttttctt cattttaatt atcatagtat ttgtttgaaa 2040
   aaaaaaaaaa aaaatttccc ttatcaatga tatccttacg attatataaa ttccttacct 2100
   aaacctatta tttgtgtaca tatatcagag tattattaca tatataacct ttttctctaa 2160
   aacaggaaaa aaaaaagaaa acgataacat gctctgccat cctttgttca ccgagcaaaa 2220
   ttaaaaacgc aaaatgaatt gtccctatga aattattaaa ggaccacatc accagactta 2280
   tctctggggg gtcctctaga aaataagtca ggtacttgcc tggactttct tccagttg   2338
<210> 14
   <211> 2338
   <212> DNA
   <213> Rattus norvegicus
<400> 14
   tcgactctag aggatcccct taagctaatc cttatgaatc cggagaaaag cggggtcttt 60
   taactcaata aaattttccg aaatcctttt tcctacgcgt tttcttcggg aactagatag 120
   gtggctcttc cacctgtttt tccatcattt tagtttttcg caagccatgc gtgccttttc 180
   gtttttgcga tggcgaacga gggctggaaa aattaacggt acgccgccta acgatagtaa 240
   taggccacgc aactggcgtg gacgacaaca ataagtcgcc cattttttat gttttcaaaa 300
   cctagcaacc cccaccaaac ttgtcatcgt tcccggattc acaaatgata taaaaagcga 360
   ttacaattct acattctaac cagatttgag atttcctctt tctcaattcc tcttatatta 420
   gattataaga acaacaaatt aaattacaaa aagacttata aagcaacata atgtctgaat 480
   tcagcaagaa ggtgacgggc cgccttatgt tggccgtggg aggggcagtg ctcggatccc 540
   tgcagttcgg ctataacacc ggtgtcatca acgcccccca gaaggtaatt gaggagttct 600
   acaatcaaac atggaaccac cgctatggag agtccatccc atccaccaca ctcaccacac 660
   tctggtctct ctccgtgatg atcttctctg tcgggggcat gattggttcc ttctctgtgg 720
   gcctctttgt taatcgcttt ggcaggcgga actccatgct gatgatgaac ctgttggcct 780
   ttgtgtctgc cgtgcttatg ggtttctcca aactgggcaa gtcctttgag atgctgatcc 840
   tgggccgctt catcattgga gtgtactgtg gcctgaccac cggctttgtg cccatgtatg 900
   tgggggaggt gtcacccaca gctcttcgtg gagccctggg caccctgcac cagctgggca 960
   tcgtcgttgg gatccttatt gcccaggtgt tcggcttaga ctccatcatg ggcaatgcag 1020
   acttgtggcc tctactgctc agtgtcatct tcatcccagc cctgctacag tgtatcctgt 1080
   tgcccttctg ccctgagagc ccccgcttcc tgctcatcaa tcgtaacgag gagaaccggg 1140
   ccaagagtgt gctgaaaaag cttcgaggga cagccgatgt gacccgagac ctgcaggaga 1200
   tgaaagaaga gggtcggcag atgatgcggg agaagaaggt caccatcttg gagctgttcc 1260
   gctcacccgc ctaccgccag cccatcctca tcgccgtggt gctgcagctg tcccagcagc 1320
   tgtcgggcat caatgctgtg ttctactact caacgagcat cttcgagaag gcaggtgtgc 1380
   agcagcctgt gtatgccacc atcggctcgg gtatcgtcaa cacggccttc actgtggtgt 1440
   cgctgttcgt cgtggagcga gctggccgtc ggaccctgca tctcattggt ctggctggca 1500
   tggcgggctg tgctgtgctc atgaccatcg ccctggccct gctggagcag ctgccctgga 1560
   tgtcctatct gagtatcgtg gccatctttg gctttgtggc cttctttgaa gtaggccctg 1620
   gtcctattcc atggttcatt gtggccgagc tgttcagcca ggggccccga cctgctgctg 1680
   ttgctgtggc tggcttctct aactggacct caaacttcat cgtgggcatg tgcttccaat 1740
   atgtggagca actgtgtggc ccctacgtct tcatcatctt cacggtgctg ctggtactct 1800
   tcttcatctt cacctacttc aaagttcctg agaccaaagg ccggaccttc gatgagatcg 1860
   cttccggctt ccggcagggg ggtgccagcc agagcgacaa gacacctgag gagctcttcc 1920
   accctctggg ggctgactcc caagtgtaat cgatttgaag tgagacgctc catcatctct 1980
   cttaattttt catgactgac gttttttctt cattttaatt atcatagtat ttgtttgaaa 2040
   aaaaaaaaaa aaaatttccc ttatcaatga tatccttacg attatataaa ttccttacct 2100
   aaacctatta tttgtgtaca tatatcagag tattattaca tatataacct ttttctctaa 2160
   aacaggaaaa aaaaaagaaa acgataacat gctctgccat cctttgttca ccgagcaaaa 2220
   ttaaaaacgc aaaatgaatt gtccctatga aattattaaa ggaccacatc accagactta 2280
   tctctggggg gtcctctaga aaataagtca ggtacttgcc tggactttct tccagttg   2338
<210> 15
   <211> 6360
   <212> DNA
   <213> Vektor
<400> 15
   cgtaggaaca atttcgggcc cctgcgtgtt cttctgaggt tcatctttta catttgcttc 60
   tgctggataa ttttcagagg caacaaggaa aaattagatg gcaaaaagtc gtctttcaag 120
   gaaaaatccc caccatcttt cgagatcccc tgtaacttat tggcaactga aagaatgaaa 180
   aggaggaaaa tacaaaatat actagaactg aaaaaaaaaa agtataaata gagacgatat 240
   atgccaatac ttcacaatgt tcgaatctat tcttcatttg cagctattgt aaaataataa 300
   aacatcaaga acaaacaagc tcaacttgtc ttttctaaga acaaagaata aacacaaaaa 360
   caaaaagttt ttttaatttt aatcaaaaag ttaacatgca tcaccatcac catcacacta 420
   gtggatcccc cgggctgcag gaattcgata tcaagcttat cgataccgtc gacctcgagt 480
   catgtaatta gttatgtcac gcttacattc acgccctccc cccacatccg ctctaaccga 540
   aaaggaagga gttagacaac ctgaagtcta ggtccctatt tattttttta tagttatgtt 600
   agtattaaga acgttattta tatttcaaat ttttcttttt tttctgtaca gacgcgtgta 660
   cgcatgtaac attatactga aaaccttgct tgagaaggtt ttgggacgct cgaaggcttt 720
   aatttgcggc cggtacccaa ttcgccctat agtgagtcgt attacgcgcg ctcactggcc 780
   gtcgttttac aacgtcgtga ctgggaaaac cctggcgtta cccaacttaa tcgccttgca 840
   gcacatcccc ctttcgccag ctggcgtaat agcgaagagg cccgcaccga tcgcccttcc 900
   caacagttgc gcagcctgaa tggcgaatgg cgcgacgcgc cctgtagcgg cgcattaagc 960
   gcggcgggtg tggtggttac gcgcagcgtg accgctacac ttgccagcgc cctagcgccc 1020
   gctcctttcg ctttcttccc ttcctttctc gccacgttcg ccggctttcc ccgtcaagct 1080
   ctaaatcggg ggctcccttt agggttccga tttagtgctt tacggcacct cgaccccaaa 1140
   aaacttgatt agggtgatgg ttcacgtagt gggccatcgc cctgatagac ggtttttcgc 1200
   cctttgacgt tggagtccac gttctttaat agtggactct tgttccaaac tggaacaaca 1260
   ctcaacccta tctcggtcta ttcttttgat ttataaggga ttttgccgat ttcggcctat 1320
   tggttaaaaa atgagctgat ttaacaaaaa tttaacgcga attttaacaa aatattaacg 1380
   tttacaattt cctgatgcgg tattttctcc ttacgcatct gtgcggtatt tcacaccgca 1440
   tagggtaata actgatataa ttaaattgaa gctctaattt gtgagtttag tatacatgca 1500
   tttacttata atacagtttt ttagttttgc tggccgcatc ttctcaaata tgcttcccag 1560
   cctgcttttc tgtaacgttc accctctacc ttagcatccc ttccctttgc aaatagtcct 1620
   cttccaacaa taataatgtc agatcctgta gagaccacat catccacggt tctatactgt 1680
   tgacccaatg cgtctccctt gtcatctaaa cccacaccgg gtgtcataat caaccaatcg 1740
   taaccttcat ctcttccacc catgtctctt tgagcaataa agccgataac aaaatctttg 1800
   tcgctcttcg caatgtcaac agtaccctta gtatattctc cagtagatag ggagcccttg 1860
   catgacaatt ctgctaacat caaaaggcct ctaggttcct ttgttacttc ttctgccgcc 1920
   tgcttcaaac cgctaacaat acctgggccc accacaccgt gtgcattcgt aatgtctgcc 1980
   cattctgcta ttctgtatac acccgcagag tactgcaatt tgactgtatt accaatgtca 2040
   gcaaattttc tgtcttcgaa gagtaaaaaa ttgtacttgg cggataatgc ctttagcggc 2100
   ttaactgtgc cctccatgga aaaatcagtc aagatatcca catgtgtttt tagtaaacaa 2160
   attttgggac ctaatgcttc aactaactcc agtaattcct tggtggtacg aacatccaat 2220
   gaagcacaca agtttgtttg cttttcgtgc atgatattaa atagcttggc agcaacagga 2280
   ctaggatgag tagcagcacg ttccttatat gtagctttcg acatgattta tcttcgtttc 2340
   ctgcaggttt ttgttctgtg cagttgggtt aagaatactg ggcaatttca tgtttcttca 2400
   acactacata tgcgtatata taccaatcta agtctgtgct ccttccttcg ttcttccttc 2460
   tgttcggaga ttaccgaatc aaaaaaattt caaagaaacc gaaatcaaaa aaaagaataa 2520
   aaaaaaaatg atgaattgaa ttgaaaagct gtggtatggt gcactctcag tacaatctgc 2580
   tctgatgccg catagttaag ccagccccga cacccgccaa cacccgctga cgcgccctga 2640
   cgggcttgtc tgctcccggc atccgcttac agacaagctg tgaccgtctc cgggagctgc 2700
   atgtgtcaga ggttttcacc gtcatcaccg aaacgcgcga gacgaaaggg cctcgtgata 2760
   cgcctatttt tataggttaa tgtcatgata ataatggttt cttagtatga tccaatatca 2820
   aaggaaatga tagcattgaa ggatgagact aatccaattg aggagtggca gcatatagaa 2880
   cagctaaagg gtagtgctga aggaagcata cgataccccg catggaatgg gataatatca 2940
   caggaggtac tagactacct ttcatcctac ataaatagac gcatataagt acgcatttaa 3000
   gcataaacac gcactatgcc gttcttctca tgtatatata tatacaggca acacgcagat 3060
   ataggtgcga cgtgaacagt gagctgtatg tgcgcagctc gcgttgcatt ttcggaagcg 3120
   ctcgttttcg gaaacgcttt gaagttccta ttccgaagtt cctattctct agaaagtata 3180
   ggaacttcag agcgcttttg aaaaccaaaa gcgctctgaa gacgcacttt caaaaaacca 3240
   aaaacgcacc ggactgtaac gagctactaa aatattgcga ataccgcttc cacaaacatt 3300
   gctcaaaagt atctctttgc tatatatctc tgtgctatat ccctatataa cctacccatc 3360
   cacctttcgc tccttgaact tgcatctaaa ctcgacctct acatttttta tgtttatctc 3420
   tagtattact ctttagacaa aaaaattgta gtaagaacta ttcatagagt gaatcgaaaa 3480
   caatacgaaa atgtaaacat ttcctatacg tagtatatag agacaaaata gaagaaaccg 3540
   ttcataattt tctgaccaat gaagaatcat caacgctatc actttctgtt cacaaagtat 3600
   gcgcaatcca catcggtata gaatataatc ggggatgcct ttatcttgaa aaaatgcacc 3660
   cgcagcttcg ctagtaatca gtaaacgcgg gaagtggagt caggcttttt ttatggaaga 3720
   gaaaatagac accaaagtag ccttcttcta accttaacgg acctacagtg caaaaagtta 3780
   tcaagagact gcattataga gcgcacaaag gagaaaaaaa gtaatctaag atgctttgtt 3840
   agaaaaatag cgctctcggg atgcattttt gtagaacaaa aaagaagtat agattctttg 3900
   ttggtaaaat agcgctctcg cgttgcattt ctgttctgta aaaatgcagc tcagattctt 3960
   tgtttgaaaa attagcgctc tcgcgttgca tttttgtttt acaaaaatga agcacagatt 4020
   cttcgttggt aaaatagcgc tttcgcgttg catttctgtt ctgtaaaaat gcagctcaga 4080
   ttctttgttt gaaaaattag cgctctcgcg ttgcattttt gttctacaaa atgaagcaca 4140
   gatgcttcgt tcaggtggca cttttcgggg aaatgtgcgc ggaaccccta tttgtttatt 4200
   tttctaaata cattcaaata tgtatccgct catgagacaa taaccctgat aaatgcttca 4260
   ataatattga aaaaggaaga gtatgagtat tcaacatttc cgtgtcgccc ttattccctt 4320
   ttttgcggca ttttgccttc ctgtttttgc tcacccagaa acgctggtga aagtaaaaga 4380
   tgctgaagat cagttgggtg cacgagtggg ttacatcgaa ctggatctca acagcggtaa 4440
   gatccttgag agttttcgcc ccgaagaacg ttttccaatg atgagcactt ttaaagttct 4500
   gctatgtggc gcggtattat cccgtattga cgccgggcaa gagcaactcg gtcgccgcat 4560
   acactattct cagaatgact tggttgagta ctcaccagtc acagaaaagc atcttacgga 4620
   tggcatgaca gtaagagaat tatgcagtgc tgccataacc atgagtgata acactgcggc 4680
   caacttactt ctgacaacga tcggaggacc gaaggagcta accgcttttt tgcacaacat 4740
   gggggatcat gtaactcgcc ttgatcgttg ggaaccggag ctgaatgaag ccataccaaa 4800
   cgacgagcgt gacaccacga tgcctgtagc aatggcaaca acgttgcgca aactattaac 4860
   tggcgaacta cttactctag cttcccggca acaattaata gactggatgg aggcggataa 4920
   agttgcagga ccacttctgc gctcggccct tccggctggc tggtttattg ctgataaatc 4980
   tggagccggt gagcgtgggt ctcgcggtat cattgcagca ctggggccag atggtaagcc 5040
   ctcccgtatc gtagttatct acacgacggg gagtcaggca actatggatg aacgaaatag 5100
   acagatcgct gagataggtg cctcactgat taagcattgg taactgtcag accaagttta 5160
   ctcatatata ctttagattg atttaaaact tcatttttaa tttaaaagga tctaggtgaa 5220
   gatccttttt gataatctca tgaccaaaat cccttaacgt gagttttcgt tccactgagc 5280
   gtcagacccc gtagaaaaga tcaaaggatc ttcttgagat cctttttttc tgcgcgtaat 5340
   ctgctgcttg caaacaaaaa aaccaccgct accagcggtg gtttgtttgc cggatcaaga 5400
   gctaccaact ctttttccga aggtaactgg cttcagcaga gcgcagatac caaatactgt 5460
   ccttctagtg tagccgtagt taggccacca cttcaagaac tctgtagcac cgcctacata 5520
   cctcgctctg ctaatcctgt taccagtggc tgctgccagt ggcgataagt cgtgtcttac 5580
   cgggttggac tcaagacgat agttaccgga taaggcgcag cggtcgggct gaacgggggg 5640
   ttcgtgcaca cagcccagct tggagcgaac gacctacacc gaactgagat acctacagcg 5700
   tgagctatga gaaagcgcca cgcttcccga agggagaaag gcggacaggt atccggtaag 5760
   cggcagggtc ggaacaggag agcgcacgag ggagcttcca gggggaaacg cctggtatct 5820
   ttatagtcct gtcgggtttc gccacctctg acttgagcgt cgatttttgt gatgctcgtc 5880
   aggggggcgg agcctatgga aaaacgccag caacgcggcc tttttacggt tcctggcctt 5940
   ttgctggcct tttgctcaca tgttctttcc tgcgttatcc cctgattctg tggataaccg 6000
   tattaccgcc tttgagtgag ctgataccgc tcgccgcagc cgaacgaccg agcgcagcga 6060
   gtcagtgagc gaggaagcgg aagagcgccc aatacgcaaa ccgcctctcc ccgcgcgttg 6120
   gccgattcat taatgcagct ggcacgacag gtttcccgac tggaaagcgg gcagtgagcg 6180
   caacgcaatt aatgtgagtt acctcactca ttaggcaccc caggctttac actttatgct 6240
   tccggctcct atgttgtgtg gaattgtgag cggataacaa tttcacacag gaaacagcta 6300
   tgaccatgat tacgccaagc gcgcaattaa ccctcactaa agggaacaaa agctggagct 6360
<210> 16
   <211> 24
   <212> DNA
   <213> Rattus norvegicus
<400> 16
   ctttctcaat tcctcttata ttag 24
<210> 17
   <211> 36
   <212> DNA
   <213> Rattus norvegicus
<400> 17
   cccgacagag aagatcatca cggagagaga ccagag 36
<210> 18
   <211> 24
   <212> DNA
   <213> Rattus norvegicus
<400> 18
   aacgtcagtc atgaaaaatt aaga 24

## Beschreibung

Die Erfindung betrifft einen Stamm der Hefe *Saccharomyces cerevisiae,* welcher durch Deletion der genomischen Sequenzen keine Hexosetransporter mehr ausbildet und in Folge dessen sich auf Substraten mit Hexosen als einziger Kohlenstoffquelle nicht mehr vermehren kann, wobei dessen Fähigkeit sich auf einem Substrat mit einer Hexose als einziger Kohlenstoffquelle zu vermehren wiederhergestellt wird, wenn er ein GLUT4-Gen zur Expression bringt.

Die meisten heterotrophen Zellen transportieren Glukose über spezielle Transporterproteine ins Zellinnere. Bei den verschiedenen Organismen haben sich unterschiedliche Mechanismen herausgebildet, die den Glukosetransport vermitteln, also Protonen-Symportsysteme, Na⁺-Glukosecotransporter, bindungsproteinabhängige Systeme, Phosphotransferasesysteme sowie Systeme für die erleichterte Diffusion. Bei den Eukaryonten vermittelt eine Familie von Glukosetransportern, die bei Säugetieren von den *GLUT*-Genen und bei *Saccharomyces cerevisiae* von den *HXT*-Genen codiert werden, die Glukoseaufnahme über erleichterte Diffusion. Diese Transporter zählen zu einer größeren Zuckertransport-Superfamilie und sind durch das Vorliegen 12 transmembranöser Helices und mehrerer konservierter Aminosäurereste und - motive gekennzeichnet.

Der Glukosetransport bei Säugetieren war Gegenstand zahlreicher Untersuchungen, da die Kenntnis der Vorgänge bei Krankheiten, die mit einer defekten Glukosehomöostase assoziiert sind, wie zum Beispiel Diabetes mellitus oder Fanconi-Bickel-Syndrom, von großer Wichtigkeit ist. Bis zum gegenwärtigen Zeitpunkt sind acht Glukosetranspörter (GLUT1 bis GLUT5, GLUT8, GLUT9/SLC2A9, GLUT9 (GenBank Aufnahme-Nr. Y17803)) identifiziert worden, welche zur erleichterten Glukoseaufnahme beitragen. Zu den Schlüsselrollen dieser Transporter zählen die Aufnahme von Glukose in verschiedene Gewebe, ihre Speicherung in der Leber, ihre insulinabhängige Aufnahme in die Muskelzellen und Adipozyten sowie die Glukose-Messung durch die β-Zellen des Pankreas.

GLUT1 vermittelt den Glukosetransport in die Erythrozyten und durch die Blut-Hirn-Schranke, wird jedoch auch in vielen anderen Geweben exprimiert, während GLUT4 auf insulinabhängige Gewebe, in erster Linie auf Muskel- und Fettgewebe beschränkt ist. Bei diesen insulinabhängigen Geweben stellt die Kontrolle des Targeting von GLUT4-Transportern in intrazelluläre Kompartimente oder Plasmamembrankompartimente einen wichtigen Mechanismus für die Regulierung der Glukoseaufnahme dar. In Gegenwart von Insulin wird intrazelluläres GLUT4 auf die Plasmamembran zurückverteilt, um die Glukoseaufnahme zu erleichtern. GLUT1 wird in diesen insulinabhängigen Geweben ebenfalls exprimiert, und seine Verteilung in der Zelle wird ebenfalls von Insulin beeinflußt, jedoch weniger stark.

Darüberhinaus wird die relative Wirksamkeit, mit der GLUT1 oder GLUT4 den Zuckertransport katalysieren, nicht nur von dem Ausmaß des Targetings jedes Transporters an die Zelloberfläche bestimmt, sondern auch von ihren kinetischen Eigenschaften.

Die Tatsache, daß unterschiedliche Glukosetransporter-Isoformen koexprimiert werden sowie der rasche Glukosemetabolismus hat Untersuchungen bezüglich der Rolle und der genauen Eigenschaften jeder Glukosetransporter-Isoform in diesen insulinabhängigen Geweben kompliziert gestaltet. Um diese Probleme zu lösen, wurden heterologe Expressionssysteme wie Xenopus-Oozyten, Gewebekulturzellen, Insektenzellen und Hefezellen verwendet. Es stellte sich jedoch heraus, daß Schwierigkeiten bei diesen Systemen auftraten: zu schwache Aktivität der heterolog exprimierten Transporter, eigene Glukosetransporter bei diesen Systemen, die intrazelluläre Retention eines beträchtlichen Teils der Transporter, oder sogar die Produktion inaktiver Transporter.

Kasahara et. al. (Biochim. et Biophys. Acta Bd. 1324, Nr. 1, 1997, Seiten 111-119) beschreibt die Expression von Glut4 in S. cerevisiae. Der verwendeten Stamm LBY416 ist deletiert in zwei Glucose-Transportern HXT2, und SNF3. Der Stamm wird auch in Kruckeberg et al, (Molecular and Cellular BiologyBd. 10 Nr. 11, 1990, Seiten 5903-5913) beschrieben. Es wird gezeigt, dass LBY416 im Glukostransport defizient ist und nicht auf niedrig-Glukose Medium wächst.

Bislang ist noch kein Organismus bekannt, der außer einem heterologen und funktionellen Glut4-Glukosetransportprotein kein weiteres insbesondere intrinsisches Hexosetransportprotein exprimiert. Dies führt zu einer Reihe von Nachteilen bei der Suche nach Verbindungen, welche die Transporteigenschaften des Glut4-Proteins verändern können. Solche Verbindungen wären von großem Interesse als Bestandteile von Arzneimitteln, da bekannt ist, daß Glut4 bei der Absenkung der Blutglukosekonzentration im Zusammenspiel mit Insulin und anderen Faktoren eine wichtige Rolle spielt. Mit Hilfe eines Organismus, der ein funktionelles GIut4-Transportprotein exprimiert, hätte man die Möglichkeit nach Verbindungen zu suchen, die den Glut4-Transporter direkt beeinflußen. Die Nebenwirkungen solcher Verbindungen wären geringer, da keine Nebeneffekte vermittelt über Signalfaktoren auftreten würden. Außerdem wären die Handhabung und Bereitstellung des Materials im Falle eines zur Verfügung stehenden Hefestammes sehr erleichtert. Als Aufgabe der Erfindung wird deshalb die Bereitstellung eines Hefestammes angesehen, der ein funktionelles Glut4-Protein exprimiert.

Die Erfindung betrifft einen Stamm der Hefe *Saccharomyces cerevisiae,* welcher sich auf Substraten mit Hexosen als einziger Kohlenstoffquelle nicht mehr vermehren kann, wobei dessen Fähigkeit sich auf einem Substrat mit einer Hexose als einziger Kohlenstoffquelle zu vermehren wiederhergestellt wird, wenn er ein GLUT4-Gen zur Expression bringt. Solch ein Stamm kann beispielsweise durch Mutation oder Deletion der entsprechenden genomischen Sequenzen hergestellt werden. Hexosen soll als Bezeichnung für Aldosen mit 6 Kohlenstoffatomen wie Glukose, Galaktose oder Mannose sowie für Ketosen mit 6 Kohlenstoffatomen wie Fruktose oder Sorbose verwendet werden.

Die Erfindung betrifft weiterhin einen Stamm der Hefe *Saccharomyces cerevisiae* wie eben beschrieben, wobei dieser Stamm ein GLUT4-Gen enthält.

Bei der Hefe *Saccharomyces cerevisiae* sind 17 Hexosetransporter und zusätzlich drei Maltosetransporter bekannt, die, sofern sie stark genug exprimiert werden, in der Lage sind, Hexosen in die Hefe zu transportieren. Bekannt ist ein Stamm, dem durch Deletion sämtliche Transporter, die zur Hexoseaufnahme geeignet sind, entfernt wurden. Dieser Stamm enthält lediglich noch die beiden Gene MPH2 und MPH3, die zu Maltosetransportproteinen homolog sind. Die beiden Gene MPH2 und MPH3 werden bei Anwesenheit von Glukose im Medium reprimiert. Herstellung und Charakterisierung dieses Hefestammes ist in Wieczorke et al., FEBS Lett. 464, 123 - 128 (1999) beschrieben. Dieser Stamm ist nicht in der Lage, sich auf einem Substrat mit einer Glukose als einziger Kohlenstoffquelle zu vermehren. Aus diesem Stamm können Mutanten selektiert werden, die ausgehend von einem entsprechenden Vektor Glut1 funktionell exprimieren (Stamm hxt fgy1-1). Transformiert man in den Hefestamm hxt fgy1-1 einen Plasmidvektor, welcher ein GLUT4-Gen unter Kontrolle eines Hefepromotors trägt, wird dennoch nur sehr wenig Glukose transportiert. Die funktionelle Expression von Glut4 erfordert weitere Anpassungen dieses Hefestammes, um einen signifikanten Glukosetransport mittels Glut4 zu ermöglichen. Solche Hefestämme, die Glukose mittels eines einzigen Glukosetransporters Glut4 in die Zellen aufnehmen, lassen sich auf Substraten mit Glukose als einziger Kohlenstoffquelle isolieren. Dazu wird ein Hefestamm hxt fgy1-1 der ein Glut4-Gen unter funktioneller Kontrolle eines Hefepromotors trägt, transformiert. Diese so transformierten Hefezellen werden auf einem Nährmedium ausgebracht, welches Glukose als einzige Kohlenstoffquelle enthält, und werden darauf inkubiert. Nach einigen Tagen der Inkubation bei beispielsweise 30°C beobachtet man Wachstum von vereinzelten Kolonien. Eine dieser Kolonien wird isoliert. Entfernt man aus dieser Kolonie das Hefeplasmid, unterbleibt die Vermehrung auf dem Nährmedium mit Glukose als einziger Kohlenstoffquelle. Wird diesem Stamm, der nun kein Vektorplasmid mehr enthält, wiederum ein Hefevektor, der ein GLUT4-Gen unter funktioneller Kontrolle eines Hefepromotors trägt, durch Transformation zugeführt, dann ist dieser Stamm wiederum in der Lage, sich auf einem Medium mit Glukose als einziger Kohlenstoffquelle zu vermehren. Die Herstellung eines Stammes von *Saccharomyces cerevisiae,* welcher die Glukoseaufnahme mittels eines Glut4-Transporters ermöglicht, wird in den Beispielen ausführlich beschrieben. Dieser Stamm exprimiert keine hefeeigenen Transporter für Hexosen und ist in der Lage mittels eines beispielsweise durch Transformation eingeführten Gens für einen Glut4-Transporter Hexosen insbesondere Glukose in die Zelle aufzunehmen. Hefestämme mit dieser Eigenschaft wurden unter der Nummer DSM 14035, DSM 14036 oder DSM 14037 bei der Deutschen Sammlung von Mikroorganismen und Zellkulturen GmbH, (DSMZ) in Braunschweig gemäß dem Budapester Vertrag über die internationale Anerkennung der Hinterlegung von Mikroorganismen für die Zwecke von Patentverfahren hinterlegt (Tabelle 1).

Dem Fachmann sind Expressionspromotoren der Hefe bekannt. Solche sind beispielsweise der SOD1-Promotor (Superoxiddismutase), ADH-Promotor (Alkoholdehydrogenase), der Promotor für das Gen der sauren Phosphatase, HXT2-Promotor (Glukosetransporter 2), HXT7-Promotor (Glukosetransporter 7), GAL2-Promotor (Galaktosetransporter) und andere. Das Konstrukt bestehend aus einem Expressionspromotor einer Hefe und einem GLUT4-Gen ist für den Zweck der Expression Bestandteil eines Hefevektors. Dieser Hefevektor kann zur Durchführung der Expression als selbstreplizierendes Partikel unabhängig vom Genom der Hefe vorliegen oder stabil in das Genom der Hefe integriert sein. Als Hefevektor eignet sich grundsätzlich jede Polynukleotidsequenz, welche in einer Hefe vermehrt werden kann. Als Hefevektoren können insbesondere Hefeplasmide oder künstliche Hefechromosomen (Yeast Artificial Chromosomes) verwendet werden. Hefevektoren enthalten in der Regel einen "origin of replication" (2µ, ars) für die Einleitung der Replikation sowie einen Selektionsmarker, der üblicherweise aus einem Auxotrophiemarker oder einem Antibiotikumresistenzgen besteht. Einem Fachmann als Hefevektoren bekannt sind beispielsweise pBM272, pCS19, pEMBCYe23, pFL26, pG6, pNN414, pTV3 oder andere. Grundsätzlich kann das GLUT4-Gen jeder Spezies zur Expression gebracht werden. Bevorzugt wird ein GLUT4-Gen aus Mensch, Maus oder Ratte zur Expression gebracht. Die Polynukleotid- und Aminosäuresequenzen für Glut4 sind zugänglich beispielsweise über folgende Einträge in Genbank: M20747 (cDNA; Mensch), EMBL:D28561 (cDNA; Ratte), EMBL:M23382 (cDNA; Maus), Swissprot:P14672 (Protein; Mensch), Swissprot:P19357 (Protein; Ratte) und Swissprot:P14142 (Protein; Maus). Besonders bevorzugt wird das GLUT4-Gen mittels des Vektors YEp4H7-HsGlut4 (SEQ ID Nr. 9) zur Expression gebracht. Das GLUT4-Gen dieses Vektors ist menschlichen Ursprungs. Die Herstellung eines Hefevektors enthaltend ein GLUT4-Gen zur Expression in Zellen ist dem Fachmann geläufig. In den Beispielen wird die Herstellung solch eines Vektors beschrieben. Ein Hefevektor enthaltend ein Gen zur Expression wird, damit es zur Expression gebracht werden kann, durch Transformation in die Hefe eingeführt. Dazu eignen sich beispielsweise Methoden wie die Elektroporation oder die Inkubation kompetenter Zellen durch Vektor-DNA. Die Transformation ist eine dem Fachmann geläufige Technik zur Einführung von Fremd-DNA insbesondere von Plasmiden oder Vektoren in Mikroorganismen wie Hefen oder Bakterien. In der einem Fachmann bekannten Methodensammlung "Methods in Yeast Genetics, 1997: A Cold Spring Harbor Laboratory Course Manual; Adams Alison (Edt.); Cold Spring Harbor Laboratory; ISBN: 0-87969-508-0" finden sich ausführliche Protokolle zu Transformation von Hefen, Hefevektoren, Selektion von Mutanten der Hefe oder die Expression von Proteinen in Hefen. Der Nachweis erfolgter Expression des GLUT4-Gens in einer Hefe dieser Erfindung kann insbesondere durch Northern-Blotting, Western-Blotting, Glukoseaufnahmestudien sowie Glukoseverwertungsstudien oder andere Methoden geführt werden. Beim Northern-Blotting wird isolierte RNA des zu untersuchenden Organismus auf einen Träger wie beispielsweise Nitrozellulose aufgebracht und fixiert, sowie anschließend durch Inkubation dieses mit der RNA des Organismus versehenen Trägers mit radioaktiv oder über einen Fluoreszenzfarbstoff markierter DNA einer GLUT4-Polynukleotidsequenz detektiert. Die Expression von GLUT4-mRNA in einer Hefe dieser Erfindung erkennt man am Auftreten geschwärzter Banden. Im Vergleich dazu können mit der RNA einer sonst gleichen Hefe, die aber nicht mit einem GLUT4-haltigen Expressionsvektor transformiert wurde, keine geschwärzten Banden nachgewiesen werden. Beim Western-Blotting erfolgt der Nachweis des exprimierten Proteins nach Aufbringen eines Proteinextrakts des zu untersuchenden Organismus auf einen Membranträger wie Nitrozellulose über Antikörper. Antikörper für das Glut4-Protein sind beispielsweise erhältlich von Alpha Diagnostic International, Inc., 5415 Lost Lane, San Antonio, TX 78238 USA. Über diesen Anbieter können auch die zum Nachweis des gebundenen Antikörpers erforderlichen Testsysteme bezogen werden. Der Nachweis des exprimierten Glut4-Proteins wird im Vergleich zu einem sonst gleichen Hefestamm geführt, der kein Glut4-Protein enthält. Bei Glukoseaufnahmestudien wird dem zu untersuchenden Organismus radioaktiv markierte Glukose als einzige Kohlenstoffquelle angeboten. Die Hefe mit Glut4 als einzigem Glukosetransporter nimmt im Gegensatz zu einem sonst gleichen Kontrollstamm, der keinen Glut4 Transporter enthält, diese radioaktiv markierte Glukose ins Zellinnere auf. Die Glukoseverwertung kann auf Nährmedien getestet werden, die Glukose als einzige Kohlenstoffquelle enthalten. Der Hefestamm mit einem Glut4-Transportprotein als einzigem Glukosetransporter ist im Unterschied zur sonst gleichen Kontrolle ohne Glut4-Transporter in der Lage, sich im Nährmedium mit Glukose als einziger Kohlenstoffquelle zu vermehren. Diese eben erwähnten Methoden sind dem Fachmann geläufig. Ausführliche Beschreibungen finden sich beispielsweise in "Current Protocols in Molecular Biology; Edited by: F.M. Ausubel, R. Brent, R. E. Kingston, D. m. Moore, J. G. Seidman, J. A. Smith, K. Struhl; published by: John Wiley & Sons; 2000 (currently updated)".

Die Erfindung betrifft bevorzugt einen Stamm der Hefe *Saccharomyces cerevisiae* wobei ein Glut4-Gen aus Mensch, Maus oder Ratte zur Expression gebracht wird.

In einer weiteren bevorzugten Ausführungsform bezieht sich die Erfindung auf einen oder mehrere der Stämme der Hefe *Saccharomyces cerevisiae,* welcher beispielsweise unter dem Aktenzeichen DSM 14038, DSM 14039 oder DSM 14040 bei der Deutschen Sammlung von Mikroorganismen und Zellkulturen GmbH, Braunschweig hinterlegt wurden. Diese Stämme sind in Tabelle 1 aufgelistet. Diese Liste enthält die Angaben zu den verwendeten Hefestämmen, in welche die Plasmide transformiert wurden, den Plasmiden sowie den Anzuchtbedingungen für diese Hefen.

Im folgenden wird die Herstellung eines Stammes der Hefe *Saccharomyces cerevisiae* dieser Erfindung beschrieben. Eine solche ist erhältlich durch folgende Verfahrensschritte:
a) Bereitstellung einer Hefe, die sich auf Substraten mit Hexosen als einziger Kohlenstoffquelle nicht mehr vermehren kann;
b) Transformation der Hefe aus a) durch ein Plasmid, welches ein GLUT4-Gen umfaßt, welches unter funktioneller Kontrolle eines in Hefe exprimierbaren Promotors steht;
c) Ausbringen eines Stammes nach Transformation gemäß b) auf einem Medium, welches eine Hexose als einzige Kohlenstoffquelle enthält;
d) Selektion eines Stammes nach Ausbringen gemäß c), der sich auf diesem Medium vermehrt und der die Hexoseaufnahme mittels des Glut-4 Gens unterstützf.

Die Erfindung betrifft ebenso die Vermehrung eines solchen Stammes.

Zur Bereitstellung einer Hefe dieser Erfindung wird in einem ersten Schritt ein Stamm einer Hefe *Saccharomyces cerevisiae* isoliert , welcher sich auf Substraten mit Hexosen als einziger Kohlenstoffquelle nicht mehr vermehren kann, wobei dessen Fähigkeit sich auf einem Substrat mit einer Hexose als einziger Kohlenstoffquelle zu vermehren wiederhergestellt wird, wenn er ein Glut4-Gen, zur Expression bringt. Dies kann durch Mutation oder Deletion der entsprechenden genomischen Sequenzen, welche für die Hexosetransporter codieren, geschehen. Die Bereitstellung erfordert weiterhin die Vermehrung dieser Hefe. Die Vermehrung erfolgt mittels Standardmethoden der Mikrobiologie in geeigneten Medien. Geeignete Medien zur Vermehrung einer Hefe sind beispielsweise Vollmedien insbesondere YPD-Medium (Yeast extract/Peptone/Dextrose-Medium) oder selektive Medien und andere. Die Hefe-Zellen werden in diesen Nährmedien vermehrt, nach der Vermehrung durch Zentrifugation vom Medium abgetrennt und für die Zwecke des Verfahrens in einem wäßrigen Lösungsmittel enthaltend unter anderem Puffersubstanzen, Salze oder andere Zusätze in eine wäßrige Suspension gebracht. Angaben zu Vermehrung von Hefen findet der Fachmann im bereits erwähnten "Methods in Yeast Genetics, 1997: A Cold Spring Harbor Laboratory Course Manual; Adams Alison (Edt.); Cold Spring Harbor Laboratory; ISBN: 0-87969-508-0".

Zur Herstellung eines Stammes der Hefe wie vorstehend beschrieben kann zur Transformation ein GLUT4-Gen, welches unter funktioneller Kontrolle eines in Hefe exprimierbaren Promotors steht, verwendet werden.

Zur Durchführung der Transformation sei auf das vorstehend bereits erwähnte "Methods in Yeast Genetics" verwiesen.

Für die Herstellung kann auch ein GLUT4-Gen aus Mensch, Maus oder Ratte verwendet werden. Zur Transformation kann ein GLUT4-Gen verwendet werden, welches in einer Polynukleotidsequenz gemäß SEQ ID Nr. 9 oder 10 enthalten ist. SEQ ID Nr. 9 offenbart die Polynukleotidsequenz des Hefevektors Yep4H7-HsGLUT4. Dieser Vektor enthält eine Polynukleotidsequenz unter funktioneller Kontrolle des HXT7-Promotors, welche für die Aminosäuresequenz des menschlichen GLUT4-Gens codiert. In SEQ ID Nr. 10 ist die Polynukleotidsequenz des Vektors H2rg4g2 enthalten. Das Hefeplasmid H2rg4g2 trägt ein GLUT4-Gen der Ratte unter funktioneller Kontrolle eines HXT2-Promotors. Funktionelle Kontrolle des GLUT4-Gens durch den Promotor bedeutet, daß mittels des Promotors eine mRNA transkribiert wird; die in ein Glut4-Protein translatiert werden kann. Bezüglich der Offenbarungen der GLUT4-Sequenzen und der verwendeten Methoden sei auf das bereits vorstehend ausgeführte hierzu verwiesen.

### Z

Die Bestimmung der Menge einer Hexose, die von einem Hefestamm aufgenommem wird, kann mittels Aufnahmestudien mit radioaktiv markierter Glukose erfolgen. Dazu wird eine bestimmte Menge der Hefezellen beispielsweise eine Menge von 60 mg Naßgewicht pro ml in beispielsweise 100 µl eines Puffers suspendiert und mit einer definierten Menge von ¹⁴C- oder ³H- markierter Glukose als einziger Kohlenstoffquelle versetzt. Man inkubiert die Zellen und entnimmt zu bestimmten Zeiten definierte Mengen der Zellen. Die Bestimmung der aufgenommenen Menge an Glukose erfolgt mit Hilfe von LSC (Liquid Scintillation Counting = Flüssig-Szintillationszählung).

Die Bestimmung der Menge einer Hexose, die von einem wie eben vorstehend beschrieben bereitgestellten Hefestamm aufgenommem wird, kann aber auch mittels Wachstumstests auf Medien mit Glukose als einziger Kohlenstoffquelle erfolgen. Dazu bestimmt man die Wachstumsrate des Stammes nach Zugabe der Verbindung beispielsweise durch regelmäßige Messungen der optischen Dichte der Kultur bei 600 nm und vergleicht diesen Wert mit der Wachstumsrate eines Kontrollstammes (z.B. Wildtyphefestamm).

Bei der Hefe *Saccharomyces, cerevisiae* sind 17 Hexosetransporter und zusätzlich drei Maltosetransporter bekannt, die in der Lage sind, Hexosen in die Hefe zu transportieren. Bekannt ist ein Stamm, dem durch Deletion sämliche Transporter, die zur Hexoseaufnahme geeignet sind, entfernt wurden. Herstellung und Charakterisierung dieses Hefestammes ist in Wieczorke et al., FEBS Lett. 464, 123 - 128 (1999) beschrieben. Dieser Stamm ist nicht in der Lage, sich auf einem Substrat mit einer Hexose als einziger Kohlenstoffquelle zu vermehren. Transformiert man in solch einen Hefestamm einen Plasmidvektor, welcher ein Glut1-Gen unter Kontrolle eines Hefepromotors trägt, wird dennoch keine Glukose transportiert. Die funktionelle Expression von Glut1 erfordert weitere Anpassungen dieses Hefestammes, um den Glukosetransport mittels Glut1 zu ermöglichen. Solche Hefestämme, die Glukose mittels eines einzigen Glukosetransporters Glut1 in die Zellen aufnehmen, lassen sich auf Substraten mit Glukose als einziger Kohlenstoffquelle isolieren. Dazu wird ein Hefestamm, der keine intakten Hexose transportierenden Proteine mehr exprimiert mit einem Hefevektor, der ein GLUT1-Gen unter funktioneller Kontrolle eines Hefepromotors trägt, transformiert. Diese so transformierten Hefezellen werden auf einem Nährmedium ausgebracht, welches Glukose als einziger Kohlenstoffquelle enthält und werden darauf inkubiert. Nach einigen Tagen der Inkubation bei beispielsweise 30°C beobachtet man Wachstum von vereinzelten Kolonien. Eine dieser Kolonien wird isoliert. Entfernt man aus dieser Kolonie das Hefeplasmid, unterbleibt die Vermehrung auf dem Nährmedium mit Glukose als einziger Kohlenstoffquelle. Wird diesem Stamm, der nun kein Vektorplasmid mehr enthält, wiederum ein Hefevektor, der ein GLUT1-Gen unter funktioneller Kontrolle eines Hefepromotors trägt, durch Transformation zugeführt, dann ist dieser Stamm wiederum in der Lage, sich auf einem Medium mit Glukose als einziger Kohlenstoffquelle zu vermehren.

Polynukleotidsequenzen und Aminosäuresequenzen für Glut1 sind offenbart unter den folgenden Code-Nummern der angegebenen Datenbanken: EMBL:M20653 (cDNA; Mensch), EMBL:M13979 (cDNA; Ratte), EMBL:M23384 (cDNA; Maus), Swissprot:P11166 (Protein; Mensch), Swissprot:P11167 (Protein; Ratte), Swissprot:P17809 (Protein; Maus).

Die Vermehrung von Hefestämmen kann mittels Standardmethoden der Mikrobiologie in geeigneten Medien. Geeignete Medien zur Vermehrung einer Hefe sind beispielsweise Voll medien insbesondere YPD-Medium (Yeast extract/Peptone/Dextrose-Medium) oder selektive Medien. Die Hefe-Zellen werden in diesen Nährmedien vermehrt, nach der Vermehrung durch Zentrifugation vom Medium abgetrennt und für die Zwecke des Verfahrens in einem wäßrigen Lösungsmittel enthaltend unter anderem Puffersubstanzen, Salze oder andere Zusätze in eine wäßrige Suspension gebracht. Angaben zu Vermehrung von Hefen findet der Fachmann im bereits erwähnten "Methods in Yeast Genetics, 1997: A Cold Spring Harbor Laboratory Course Manual; Adams Alison (Edt.); Cold Spring Harbor Laboratory; ISBN: 0-87969-508-0".

### Abkürzungen

- HXT: Hexose Transporter
- ORF: Open Reading Frame
- PCR: Polymerase Chain Reaction

### Beispiele

### Vermehrung der Hefestämme

Alle in der vorliegenden Arbeit beschriebenen Hefestämme stammten vom Stamm CEN.PK2-1C *(MATa leu2-3, 112 ura3-52 trp1-289 his3-Δ1 MAL2-8^{c} SUC2)* ab. Die Herstellung eines Hefestammes mit Deletionen in den Hexose-Transportergenen (*HXT*) wurde von Wieczorke et al., FEBS Lett. 464, 123 - 128 (1999) beschrieben: EBY.18ga (*MATa Δhxt1 -17 Δgal2 Δagt1 Δstl1 leu2-3, 112 ura3-52 trp1-289 his3-Δ1 MAL2-8^{c} SUC2),* EBY.VW4000 *(MATa Δhxt1-17 Δga*/*2 Δagt1 Δmph2 Δmph3 Δstl1 leu2-3, 112 ura3-52 trp1-289 his3-Δ1 MAL2-8^{c} SUC2).* Die Medien beruhten auf 1 % Hefeextrakt und 2% Pepton (YP), während die Minimalmedien aus 0,67% Difco-Hefe-Stickstoffbasis ohne Aminosäuren (YNB) bestanden und Zusätze für Auxotrophiebedürfnisse sowie unterschiedliche Kohlenstoffquellen enthielten. Die Hefezellen wurden unter aerobischen Bedingungen bei 30°C auf einem Rundschüttler oder auf Agarplatten gezüchtet. Das Zellwachstum wurde durch Messung der optischen Dichte bei 600 nm (OD₆₀₀) verfolgt.

### Bestimmung der Glukoseaufnahme

Der Glukosetransport wurde als Aufnahme von D-[U-¹⁴C]-Glukose (Amersham) gemessen und die Kinetikparameter wurden aus Eadie-Hofstee-Graphiken bestimmt. Die Zellen wurden abzentrifugiert, mit Phosphatpuffer gewaschen und wieder in Phosphatpuffer in einer Konzentration von 60 mg (Naßgewicht) pro ml suspendiert. Die Glukoseaufnahme wurde bei Glukosekonzentrationen zwischen 0,2 und 100 mM bestimmt, und die spezifische Aktivität des Substrats bewegte sich zwischen 0,1 und 55,5 kBq µmol⁻¹. Die Zellen und die Glukoselösungen wurden 5 Minuten bei 30°C vorinkubiert. Die Glukoseaufnahme wurde durch Versetzen der Zellen mit radioaktiver Glukose gestartet. Nachdem 5 Sekunden lang inkubiert worden war, versetzte man mit 10 ml eiskaltem Stoppuffer (0,1 M KiPO₄, pH 6,5, 500 mM Glukose) und die Zellen wurden rasch auf Glasfaserfiltern ∅=24 mm, Whatman) abfiltriert. Die Filter wurden dreimal rasch mit eiskaltem Puffer gewaschen und die eingebaute Radioaktivität wurde mit einem Flüssigkeits-Szintillationszähler gemessen. Die Hemmung durch Cytochalasin B (Endkonzentration 20 µM, gelöst in Ethanol) wurde in einem 15-Sekunden-Aufnahmetest mit 50 mM bzw. 100 mM radioaktiver Glukose gemessen, nachdem die Zellen 15 Minuten lang in Gegenwart des Hemmstoffs oder nur des Lösungsmittels inkubiert worden waren.

### Konstruktion von H2rg4g2 (SEQ ID Nr. 10) und H2rg1g2 (SEQ ID Nr. 12)

H2rg4g2 und H2rg1 g2 sind DNA-Konstrukte, welche einen HXT2-Promotor (Promotor des Glukosetransportproteins 2 der Hefe) enthalten, der funktionell mit einem GLUT4- (in SEQ ID Nr. 10) oder GLUT1-Gen (in SEQ ID Nr. 12) aus der Ratte verbunden ist. Es wurde ein 0,5 kB langes *Sal*I*lEco*RI*-GAL2-*Promoterfragment der Plasmide GLUT1-pTV3 bzw. GLUT4-pTV3 (Kasahara und Kasahara, Biochem J. 315, 177 - 182 (1996); Kasahara und Kasahara, Biochim. Biophys. Acta 1324, 111 - 119 (1997)) jeweils mit einem 0,5 kB langen DNA-Fragment, das den Hefe-*HXT2-*Promoter von -452 bp bis +9 bp (Genbank: P23585) enthielt, ersetzt.

### Konstruktion von YEp4H7-HsGLUT1 (SEQ ID Nr. 11) und YEp4H7-HsGLUT4 (SEQ ID Nr. 9)

YEp4H7-HsGLUT1 und YEp4H7-HsGLUT4 sind Plasmide, in welchen ein Promotorfragment der Positionen -392 bis -1 des HXT7-Promotors (Promotor des HXT7-Gens) funktionell mit einem GLUT1- (In SEQ ID Nr. 11) oder GLUT4-Gen (in SEQ ID Nr. 9) des Menschen verbunden ist. Das Fragment des Promotors wurde verwendet, da der komplette HXT7-Promotor durch Glukose reprimiert wird. Ein 0,4 kB langes *Sac*I/*Spe*I-*MET25*-Promoterfragment aus p426MET25 (Mumberg et al., Nucleic Acids Res. 22, 5767 - 5768 (1994)) wurde durch ein 0,4 kB langes DNA-Fragment enthaltend ein *HXT7-*Promoterfragment von den Positionen -392 bis -1, das mittels PCR mit den Primern P426H7-1 (SEQ ID Nr. 1) und P426H7-2 (SEQ ID Nr. 2) aus einem HXT7-Gen (Genbank:P39004) als Matrize amplifiziert worden war, ersetzt, wodurch man das Plasmid YEp4H7 (SEQ ID Nr. 15) erhielt. Die Human-GLUT1- und GLUT4-ORF (Open Reading Frame) wurden über 10 Zyklen mittels PCR mit den Primer-Paaren HSG1-F7/T2-HSG1 (SEQ ID Nr.3,4) für Glut1 und HSG4-F7/T2-HSG4 (SEQ ID Nr. 5,6) für Glut4 sowie einer humanen GLUT1 (EMBL:M20653) und humaner GLUT4-cDNA (Genbank:M20747) als Matrizen amplifiziert. Die PCR-Produkte wurden nochmals über 10 Zyklen mit den Primern T71-ORF (SEQ ID Nr. 7) sowie T2-HSG1 (SEQ ID Nr. 4) bzw. T2-HSG4 (SEQ ID Nr. 6) amplifiziert. Stromaufwärts und stromabwärts der GLUT-ORF-Sequenzen enthalten die PCR-Endprodukte Sequenzen, die zur *HXT7-*Promoter- bzw. zur *CYC1*-Terminationsregion (iso-Cytochrome c1) des Plasmids YEp4H7 homolog sind. Sie wurden gemeinsam mit dem mit *Eco*RI linearisierten YEp4H7 in den Hefestamm EBY.F4-1 transformiert, wobei man nach homologer Rekombination in Hefe auf einem 2%igen Maltosemedium auf Uracilprototrophie selektierte.

### Expression von Ratten-GLUT1 und -GLUT4 in einem hexosetransportdefizienten Hefestamm

Die Hefe-Multiköpie-Expressionsplasmide GLUT1-pTV3e und GLUT4-pTV3e tragen die Ratten-Glukosetransportergene GLUT1 bzw. GLUT4 unter der Kontrolle des galaktoseinduzierbaren und glukosereprimierbaren Hefe-*GAL2*-Promoters. In beiden Konstrukten wurde der *GAL2*-Promoter durch den glukoseinduzierbaren Hefe-*HXT2-*Promoter ersetzt. Diese Vektoren wurden in den Hefestamm EBY.18ga *(Δhxt)* transformiert, der zur Aufnahme von keinerlei Hexosen fähig ist und daher auf Medien, die Glukose oder andere Hexosen als einzige Kohlenstoffquelle enthalten, nicht wachsen kann. Die Zellen wurden auf ein tryptophanfreies synthetisches Medium mit Maltose als Kohlenstoffquelle ausplattiert. Die Transformanten wurden mit der Stempel-Methode auf das gleiche Basalmedium ohne Maltose, jedoch mit unterschiedlichen Glukosekonzentrationen (5 mM, 10 mM, 50 mM, 100 mM) ausplattiert. Die Transformanten wuchsen auf den unterschiedlichen Glukosemedien nicht, nicht einmal bei bis zu einwöchiger Inkubation bei 30°C. Dies belegt, daß der Glut1-und Glut4-Glukosetransporter in einem normalen Stamm von *S*. *cerevisiae* die Aufnahme von Glukose nicht unterstützen.

### Aufnahme von Glukose über den Glut1 -Transporter in Hefezellen

Es zeigte sich, daß nach längerer Inkubation von Glut1-Transformanten des Stamms EBY.18ga auf einem Glukosemedium sich Kolonien (im folgenden Supressormutanten oder Supressorkolonien) vermehren konnten, die offenbar fähig wurden, Glukose aufzunehmen und zu verwerten. Deshalb plattierte man die GLUT1- und GLUT4-Transformanten auf Agarplatten aus, die ein YNB-Medium mit 10 mM Glukose als einziger Kohlenstoffquelle enthielten. Nach Bestrahlung mit UV-Licht in einer subletalen Dosis wurden die Zellen 7-14 Tage bei 30°C inkubiert. Während bei den GLUT4-Transformanten keine Suppressorkolonien erschienen, wuchsen auf den Agarplatten mit den GLUT1-Transformanten mehrere Suppressorkolonien, die zum Wachstum auf Glukose *fähig* waren. Mehrere der GLUT1-Suppressormutanten wurden mehr als 15 Generationen lang in nichtselektivem YP-Maltose-Medium gezüchtet. Alle Zellen, die ihre Plasmide verloren hatten, waren nicht länger fähig, auf Medien, die Glukose als - Kohlenstoffquelle enthielten, zu wachsen.Damit konnte gezeigt werden, daß Wachstum auf Glukose als einziger Kohlenstoffquelle von GLUT1 abhängig war. Nach der Neutransformierung des ursprünglichen Wildtyp-H2rg1 g2-Plasmids in diese Zellen erlangte einer von mehreren Hefestämmen wiederum die Fähigkeit, auf Glukose zu wachsen. Das belegt, daß dieser Stamm eine Mutation in seinem Genom enthält, die die Hemmwirkung auf funktionelle GLUT1-Expression eliminiert.

Das mutierte Allel wurde mit *fgy1-1* bezeichnet (was für "functional expression of GLUT1 in yeast" steht), und der Stamm wurde mit der Bezeichung EBY.S7 versehen.

Aus anderen Suppressormutanten wurden H2rg1 g2-Plasmide isoliert, in *E. coli* amplifiziert und in den ursprünglichen glukosetransportdefizienten Hefestamm EBY.18ga (Δ*hxt*) zurücktransformiert. Mehrere dieser Plasmide konnten ein Wachstum auf einem synthetischen Medium mit 10 mM Glukose als einziger Kohlenstoffquelle ermöglichen. Diese GLUT1-Sequenzen enthielten demnach Mutationen, die das entsprechende GLUT1-Protein in der Hefe zu einem funktionellen Glukosetransporter umwandelten. Solche Mutanten enthielten beispielsweise einen Austausch von Valin nach Methionin an der Position der Aminosäure 69 (SEQ ID Nr. 13) oder einen Austausch von Alanin nach Methionin an der Position der Aminosäure 70 (SEQ ID Nr. 14). Die Mutante gemäß SEQ ID Nr. 13 wurde im Mutantenscreening wie vorstehend beschrieben gefunden. Die Mutante gemäß SEQ ID Nr. 14 erhielt man durch in vitro Mutagenese wie im folgenden dargestellt. Das Prinzip der angewandten in vitro Mutagenese-Methode ist in Boles und Miosga (1995) beschrieben (Boles und Miosga, Curr Genet. 28, 197 - 198 (1995)). In einer ersten PCR-Reaktion wurde das Plasmid YEpH2-rGLUT1 (20 ng) als DNA-Vorlage zusammen mit den Primern seqhxt2 (SEQ ID Nr. 16) und glutmet2 (SEQ ID Nr. 17) (jeweils 100 pmol) eingesetzt (PCR-Bedingungen: 95°C 45 sec, 50°C 30 sec, 72°C 2 min, 25 Zyklen, Taq-Polymerase). Der Primer glutmet2 enthält eine gegenüber dem normalen GLUT1-Gen geänderte Basensequenz, die zu einem Austausch von Alanin nach Methionin an der Position der Aminosäure 70 von GLUT1 der Ratte führt. Das resultierende PCR-Fragment wurde mittels Agarosegelelektrophorese und anschließender Gelextraktion gereinigt. In einer zweiten PCR-Reaktion wurde das gereinigte PCR-Fragment (20 ng) zusammen mit dem Plasmid GLUT1-pTV3 (Kasahara und Kasahara, Biochem J. 315, 177 - 182 (1996)) als DNA-Vorlage (50 ng) und zusammen mit den Primern seqhxt2 und seq2gal2'(SEa ID Nr. 18) (jeweils 100 pmol) eingesetzt (PCR-Bedingungen: 95°C 45 sec, 54°C 30 sec, 72°C 2 min, 20 Zyklen, Taq-Polymerase). Da der Primer seqhxt2 nur an das Fragment der ersten PCR-Reaktion bindet, wurden in dieser zweiten PCR-Reaktion nur solche DNA-Sequenzen amplifiziert, die zu einem Austausch von Alanin nach Methionin an der Position der Aminosäure 70 führen. Das resultierende PCR-Fragment mit dem mutierten GLUT1-Gen wurde mittels Agarosegelelektrophorese und anschließender Gelextraktion gereinigt und gegen das Wildtyp GLUT1-Gen im Plasmid YEpH2-rGLUT1 ausgetauscht. Dieses Plasmid (SEQ ID Nr. 14) wurde in den glukosetransportdefizienten Hefestamm EBY.18ga *(Δhxt)* transformiert und ermöglichte ein Wachstum auf einem synthetischen Medium mit Glukose als einziger Kohlenstoffquelle.

### Stämme der Hefe Saccharomyces cerevisiae, die Glukose über den Glut4-Transporter aufnehmen

Der Stamm EBY.S7 *(Δhxt fgy1-1)* enthält offenbar eine Genommutation, nämlich *fgy1-1,* die Glut1 befähigt, in der Hefe funktionell zu werden und die Aufnahme von Glukose durch die Plasmamembran hindurch in die Zellen zu unterstützen.

Nach Transformation des Stamms EBY.S7 (*Δhxt fgy1-1)* durch H2rg4g2 konnten Supressor-Kolonien isoliert werden, die zur Vermehrung auf Medien mit Glukose als einziger Kohlenstoffquelle befähigt waren.

Neun dieser GLUT4-Suppressormutanten wurden über mehr als 15 Generationen lang in nichtselektivem YP-Maltose-Medium gezüchtet. Alle Zellen, die ihre Plasmide verloren hatten, waren ebenfalls nicht mehr fähig, auf 10 mM Glukosemedien zu wachsen, was beweist, daß das frühere Wachstum GLUT4-abhängig war. Die H2rg4g2-Plasmide wurden aus den neun Suppressorstämmen wieder isoliert, in E. *coli* amplifiziert und in den ursprünglichen glukosetransportdefizienten Hefestamm EBY.S7 zurücktransformiert. Keines der Plasmide konnte auf einem synthetischen Medium mit 10 mM Glukose als einzige Kohlenstoffquelle ein Wachstum ermöglichen. Damit wurde gezeigt, daß sie keine "aktivierte" Mutantenformen von GLUT4 enthielten. Nach der erneuten Transformation des ursprünglichen Wildtyp-H2rg4g2 -Plasmids in die neun nunmehr plasmidfreien Suppressorstämme erlangten diese alle wiederum die Fähigkeit zurück, auf Glukose wachsen zu können, und zwar im Unterschied zu Transformanten, die einen Kontrollvektor ohne Glut4-Transportproteingen enthielten. Die entsprechenden Mutationen dieses Stammes wurden *fgy4-X* (x = 1 - 9) genannt. Die mutierten Allele fgy4-X bewirken die funktionelle GLUT4-Expression eines in diesen Stämmen exprimierten GLUT4-Gens. Damit konnte die Aufgabe der Erfindung gelöst werden. Die Tabelle enthält eine Übersicht der verwendeten Hefestämme dieser Erfindung.

Die Tabelle gibt eine Übersicht der in dieser Erfindung verwendeten Hefestämme einschließlich des Genotyp, den für die Vermehrung einzuhaltenden Wachstumsbedingungen und der jeweiligen Hinterlegungsnummer bei der Deutschen Sammlung von Mikroorganismen und Zellkulturen GmbH.

## Patentansprüche

1. Stamm der Hefe *Saccharomyces cerevisiae,* welcher keine hefeeigenen Transporter für Hexosen exprimiert, **dadurch gekennzeichnet, dass** dieser Stamm ein GLUT4-Gen enthält, erhältlich durch
a) Bereitstellung einer Hefe,
b) Beseitigung der Funktion aller Hexosetransporter dieser Hefe aus a) durch Mutation oder Deletion der entsprechenden genomischen Sequenzen
c) Transformation der Hefe aus b) durch ein Plasmid, welches ein GLUT4 Gen enthält, welches unter funktioneller Kontrolle eines in Hefe exprimierbaren Promoters steht,
d) Ausbringen eines Stammes nach Transformation gemäß c) auf einem Medium, welches Glukose als einzige Kohlenstoffquelle enthält,
e) Isolierung eines Stammes nach Ausbringen gemäß d), welcher sich auf einem solchen Medium vermehrt.

2. Stamm der Hefe *Saccharomyces cerevisiae* nach Anspruch 1 wie hinterlegt bei der Deutschen Sammlung von Mikroorganismen und Zellkulturen GmbH als DSM 14035, DSM 14036 oder DSM 14037.

3. Stamm der Hefe *Saccharomyces cerevisiae* nach Anspruch 1, wobei das GLUT4-Gen aus Mensch, Maus oder Ratte stammt.

4. Stamm der Hefe *Saccharomyces cerevisiae* nach Anspruch 1 oder 3 wie hinterlegt bei der Deutschen Sammlung von Mikroorganismen und Zellkulturen GmbH als DSM 14038, DSM 14039 oder DSM 14040.

5. Stamm der Hefe *Saccharomyces cerevisiae* nach Anspruch 1, wobei zur Transformation ein Vektor, der eine Polynukleotidsequenz gemäß SEQ ID Nr. 9 oder 10 enthält, verwendet wird.

6. Verfahren verwendbar zur Identifizerung einer Verbindung, welche die Menge einer Hexose, die mittels eines GLUT4-Proteins transportiert wird, vermehrt oder vermindert enthaltend folgende Verfahrensschritte:
a) Bereitstellung eines Stammes der Hefe *Saccharomyces cerevisiae* gemäß einem oder mehreren der Ansprüche 1, 2, 3 und 4;
b) Bestimmung der Menge einer Hexose, die von diesem Stamm, der gemäß a) bereitgestellt wird, aufgenommem wird;
c) Bereitstellung einer Verbindung;
d) In-Kontakt-Bringen eines Stammes der Hefe bereitgestellt gemäß a) mit einer Verbindung bereitgestellt gemäß c)
e) Bestimmung der Menge einer Hexose, die in den Stamm der Hefe nach In-Kontakt-Bringen gemäß d) aufgenommen wird;
f) Identifizierung einer Verbindung, welche die Menge einer Hexose, die mittels eines GLUT4-Proteins transportiert wird, vermehrt oder vermindert, durch Vergleich der in den Stamm aufgenommenen Menge der Hexose vor und nach In-Kontakt-Bringen gemäß d), die gemäß b) und e) bestimmt wird.

## Claims

1. Strain of the yeast *Saccharomyces cerevisiae,* which does not express any yeast-intrinsic transporters for hexosene, **characterized in that** this strain contains a GLUT4 gene, obtainable by
a) providing a yeast,
b) eliminating the function of all hexose transporters of this yeast of a) by mutating or deleting the corresponding genomic sequences,
c) transformation of the yeast of b) by a plasmid which contains a GLUT4 gene which is under the functional control of a promoter expressable in yeast,
d) applying a strain after transformation in accordance with c) to a medium which contains glucose as the only carbon source,
e) isolating of a strain after application in accordance with d) which multiplies on such a medium.

2. Strain of the yeast *Saccharomyces cerevisiae* according to Claim 1 as deposited at the Deutsche Sammlung von Mikroorganismen und Zellkulturen GmbH as DSM 14035, DSM 14036 or DSM 14037.

3. Strain of the yeast *Saccharomyces cerevisiae* according to Claim 1, where the GLUT4 gene originates from humans, mice or rats.

4. Strain of the yeast *Saccharomyces cerevisiae* according to Claim 1 or 3 as deposited at the Deutsche Sammlung von Mikroorganismen und Zellkulturen GmbH as DSM 14038, DSM 14039 or DSM 14040.

5. Strain of the yeast *Saccharomyces cerevisiae* according to Claim 1, where a vector which contains a polynucleotide sequence as shown in SEQ ID No. 9 or 10 is used for the transformation.

6. Method which can be used for identifying a compound which increases or reduces the amount of a hexose which is transported by means of a GLUT4 protein, containing the following method steps:
a) providing a strain of the yeast *Saccharomyces cerevisiae* according to one or more of claims 1, 2, 3 and 4;
b) determining the amount of a hexose which is taken up by this strain which is provided in accordance with a);
c) providing a compound;
d) bringing a strain of the yeast provided in accordance with a) into contact with a compound provided in accordance with c);
e) determining the amount of hexose which is taken up into the strain of the yeast after the bringing-into-contact in accordance with d);
f) identifying a compound which increases or reduces the amount of a hexose which is transported by means of a GLUT4 protein by comparing the amount of the hexose taken up into the strain before and after the bringing-into-contact in accordance with d), which amount is determined in accordance with b) and e).

## Revendications

1. Souche de la levure Saccharomyces cerevisiae, qui n'exprime pas de transporteur propre à la levure pour les hexoses, **caractérisée**
**en ce que** cette souche contient un gène GLUT4 pouvant être obtenu par
a) préparation d'une levure,
b) élimination de la fonction de tous les transporteurs d'hexose de cette levure de a) par mutation ou délétion des séquences génomiques correspondantes,
c) transformation de la levure de b) par un plasmide, qui contient un gène GLUT4, qui est sous un contrôle fonctionnel d'un promoteur pouvant être exprimé dans la levure,
d) étalement d'une souche après la transformation selon c) sur un milieu qui contient du glucose comme unique source de carbone,
e) isolement d'une souche après l'étalement selon d) qui se multiplie sur un tel milieu.

2. Souche de la levure Saccharomyces cerevisiae selon la revendication 1 telle que déposée auprès de la Deutsche Sammlung von Mikroorganismen und Zellkulturen GmbH sous le numéro DSM 14035, DSM 14036 ou DSM 14037.

3. Souche de la levure Saccharomyces cerevisiae selon la revendication 1, le gène GLUT4 provenant de l'homme, de la souris ou du rat.

4. Souche de la levure Saccharomyces cerevisiae selon la revendication 1 ou 3 telle que déposée auprès de la Deutsche Sammlung von Mikroorganismen und Zellkulturen GmbH sous le numéro DSM 14038, DSM 14039 ou DSM 14040.

5. Souche de la levure Saccharomyces cerevisiae selon la revendication 1, où on utilise pour la transformation un vecteur qui contient une séquence polynucléotidique selon la SEQ ID Nr. 9 ou 10.

6. Procédé utilisable pour l'identification d'un composé qui multiplie ou réduit la quantité d'un hexose qui est transporté au moyen d'une protéine GLUT4, présentant les étapes de procédé suivantes :
a) préparation d'une souche de la levure Saccharomyces cerevisiae selon l'une ou plusieurs des revendications 1, 2, 3 et 4 ;
b) détermination de la quantité d'un hexose, qui est absorbée par cette souche, préparée selon a) ;
c) préparation d'un composé ;
d) mise en contact d'une souche de levure préparée selon a) avec un composé préparé selon c);
e) détermination de la quantité d'un hexose, qui est absorbée dans la souche de la levure après la mise en contact selon d) ;
f) identification d'un composé qui multiplie ou réduit la quantité d'un hexose, qui est transporté au moyen d'une protéine GLUT4, par la comparaison de la quantité, absorbée dans la souche, d'hexose avant et après la mise en contact selon d), qui est déterminée selon b) et e).
